# EUROPEAN PATENT APPLICATION

(11) **EP 4 468 230 A1**
(43) Date of publication of application: **27.11.2024**
(21) Application number: 24178112.9
(22) Date of filing: 24.05.2024
(51) Int. Cl.: G06Q 50/20, A61B 5/16, G06Q 20/36, G09B 19/00

(54) **VISUALIZED ENGAGEMENT PROFILE**

(30) Priority: 25.05.2023 US 202363504343 P
(71) Applicant: LearningFrequency LLC, Tucson, AZ 85737 (US)
(72) Inventor: Slavin, Amanda, Tucson (US)
(74) Representative: Pfundner, Benjamin Patrick

(57) **Abstract**

Systems and methods for dynamic evaluation and visualization of student engagement levels for a personalized learning environment. A system includes an engagement module including computing hardware of at least one processor and memory operably coupled to the at least one processor. The engagement module includes a monitoring engine configured to receive behavior data associated with a period of activity of a user, an assessment engine configured to generate an assessment based on the period of activity and determine an engagement level of the user based on the behavior data and the assessment, and a visualization engine configured to generate an avatar based on the engagement level and generate a transaction on a blockchain, the transaction indicating the user owns the avatar.

## Description

### RELATED APPLICATION

The present application claims priority of U.S. Provisional Patent Application Serial No. 63/504,343 filed on May 25, 2023 and entitled VISUALIZED ENGAGEMENT PROFILE, which is incorporated by reference herein in its entirety.

### TECHNICAL FIELD

The present disclosure relates generally to user engagement and learning. More particularly, the present disclosure relates to a dynamic platform incorporating distributed storage solutions to provide real-time determination and visualization of personalized learning profiles.

### BACKGROUND

In recent years, distributed ledgers have been increasingly incorporated into applications or other computing programs to manage data for users. For example, distributed sequential transactional databases, or blockchains, are increasingly deployed to address security challenges that are unsuitable for being addressed using traditional, centralized data storage techniques.

A blockchain is a distributed chain of block data structures accessed by a network of nodes, referred to here as a miner network. A block in the blockchain includes a plurality of record data structures known as transactions, with a (e.g., each) transaction referring to or relating to a prior transaction. For example, a blockchain can include a Merkle of hash or digest values for transactions included in the block to arrive at a hash value for the block, which is itself combined with a hash value for a preceding block to generate a chain of blocks (e.g., blockchain).

A new block of transactions is added to the blockchain by miner software, hardware, firmware or combination systems in a miner network. The miners are communicatively connected to sources of transactions and access or copy the blockchain. A miner undertakes validation of the substantive content of a transaction and adds a block of new transactions to the blockchain when a challenge is satisfied as a proof of work, typically such challenge involving a combination hash or digest for a prospective new block and a preceding block in the blockchain and some challenge criterion. The process of completing this challenge is often referred to as proof of work.

Thus, miners in the miner network can each generate prospective new blocks for addition to the blockchain. Where a miner satisfies or solves the challenge and validates the transactions in a prospective new block, the new block is added to the blockchain. Accordingly, the blockchain provides a distributed mechanism for reliably verifying a data entity.

Increasing data volumes, high cost of consensus mechanisms (e.g., proof of work), and lack of error handling tend to expose deficiencies of blockchains. For example, transactions on most blockchains are irreversible due to the immutable characteristics of the blockchain, which is problematic in cases of user error or fraud. In another example, blockchain networks often struggle to handle large transaction volumes efficiently, leading to congestion, increased confirmation times, and higher transaction fees. Conventional attempts to improve blockchain scalability often introduce trade-offs in security. Moreover, blockchains typically have slower transaction processing speeds compared to traditional centralized systems, limiting their suitability for high-throughput applications.

Accordingly, ensuring scalability to handle large transaction volumes efficiently and limiting resource intensity of proof-of-work (PoW) without sacrificing security remain significant challenges in the evolving applications of blockchains. There is a need for more dynamic and flexible implementations of blockchains to leverage ownership of personalized data across networks and systems.

### SUMMARY

Embodiments described or otherwise contemplated herein substantially meet the aforementioned needs of the industry. Embodiments described herein include systems and methods for providing secure ownership of student data, for example, through blockchain and distributing machine learning workflows. Features provided herein allow for mitigation of sensitive data transfer and reduced overhead on client devices and distributed ledgers.

By addressing inefficiencies of blockchains to allow for improved incorporation with network-enabled platforms, embodiments address the need for more flexible and scalable deployment. In illustrative embodiments, the techniques of the present disclosure are generally applied to systems and methods for real-time determination and visualization of user engagement.

In an embodiment, a system for dynamic evaluation and visualization of user engagement comprises a learning platform including a memory and at least one processor. The learning platform is configured to generate a learning profile associated with a user. The learning profile includes at an engagement profile, which defines at least two levels of engagement, and an avatar that is a visual representation of the engagement profile. The learning platform is further configured to create a transaction on a blockchain including a first copy of the learning profile and to store a second copy of the learning profile in a database. The blockchain transaction can include an indication that the user owns the learning profile. The learning platform is further configured to receive a modification request associated with the learning profile and to select at least one of the first copy of the learning profile or the second copy of the learning profile to modify based on the modification request and a determination of whether the modification request is associated with the user.

In one aspect, the learning platform selects the first copy of the learning profile based on a determination that the modification request is associated with the user. In such an example, the modification can comprise creating a second transaction on the blockchain that includes, for example, the first copy of the learning profile and an indication of a modification made to the first copy of the learning profile.

In one aspect, the learning platform selects the second copy of the learning profile based on a determination that the modification request is not associated with the user. In such an example, the modification can comprise updating the second copy of the learning profile in the database.

In one aspect, the learning platform is further configured to provide a dedicated API for the user that is configured to provide access to the first copy of the learning profile and to modify the blockchain. In a second aspect, the learning platform is further configured to provide a public API configured to provide access to the second copy of the learning profile. In implementations incorporating a dedicated APO and a public API the determination, by the learning platform, of whether the modification request is associated with the user can be based on whether the modification request is received via the dedicated API or the public API.

In one aspect, the learning platform is further configured to create a digital wallet associated with the user and the blockchain and mint a non-fungible token (NFT) of the avatar in the digital wallet. The digital wallet can be configured to provide the user with access to the NFT.

In one aspect, the learning platform is further configured to obtain behavior data associated with a period of activity of a user, determine an engagement level of the user from one of the at least two levels of engagement based on the behavior data and the engagement profile, and send an indication of the engagement level. In some implementations, the engagement level is determined based on a comparison of the behavior data to historical data of the user interacting with the learning platform. In some implementations, the behavior data is associated with a type of distraction and the engagement level is further based on the type of distraction.

In one aspect, the engagement profile can be automatically updated based on a machine learning model with a feedback component incorporating the behavior data.

In one aspect, the learning profile further includes an identifier associated with the user. In such examples, the learning profile can be only associated with the user via the identifier (e.g., sensitive information is not included in the learning profile).

In one aspect, the learning platform is further configured to provide an assessment; and receive, based on the assessment, user preference data indicative of at least one user preference. In such examples, the learning profile can be generated based on the user preference data.

In one aspect, the avatar can be updated based on the modification request.

In one aspect, the blockchain is configured to store a historical record of modifications to the learning profile. In such examples, the database can be configured to only store a copy of the most-recent learning profile (e.g., to reduce the amount of stored information).

In one aspect, the learning platform is further configured to generate a second learning profile associated with the user, wherein the learning profile is associated with a first learning environment and the second learning profile is associated with a second learning environment.

In an embodiment, a method for dynamic evaluation and visualization of user engagement comprises receiving user preference data associated with a user; generating, based on the user preference data, a learning profile associated with the user, the learning profile including at an engagement profile and an avatar, wherein the engagement profile defines at least two levels of engagement; creating a transaction on a blockchain, the transaction including a first copy of the learning profile and an indication that the user owns the learning profile; storing a second copy of the learning profile in a database; providing a dedicated API for the user, wherein the dedicated API for the user is configured to provide access to the first copy of the learning profile and to modify the blockchain; providing a public API, wherein the public API is configured to provide access to the second copy of the learning profile; receiving a modification request associated with the learning profile; and selecting at least one of the first copy of the learning profile or the second copy of the learning profile to modify based on whether the modification request was received via the dedicated API or the public API.

In one aspect, the user preference data includes an indication of a selected trait from a plurality of predefined traits. In such implementations, the avatar (e.g., a feature of the avatar) can be based on the selected trait.

In one aspect, the method further comprises creating a digital wallet associated with the user and the blockchain and minting a non-fungible token (NFT) of the avatar in the digital wallet. In such implementations, the digital wallet is configured to provide the user with access to the NFT.

In one aspect, the method further comprises obtaining behavior data associated with a period of activity of a user; determining an engagement level of the user from one of the at least two levels of engagement based on the behavior data and the engagement profile; and sending an indication of the engagement level.

In one aspect, the method further comprises updating the engagement profile based on a machine learning model with a feedback component incorporating the behavior data.

In an embodiment, a system for dynamic evaluation and visualization of user engagement comprises a learning platform including a memory and at least one processor. The learning platform is configured to receive user preference data associated with a user; generate, based on the user preference data, a learning profile associated with the user, the learning profile including at an engagement profile and an avatar, wherein the engagement profile defines at least two levels of engagement; create a transaction on a blockchain, the transaction including a first copy of the learning profile and an indication that the user owns the learning profile; store a second copy of the learning profile in a database; obtain behavior data associated with a period of activity of a user; determine an engagement level of the user from one of the at least two levels of engagement based on the behavior data and the engagement profile; and generate a recommendation to improve engagement based on the determined engagement level.

In an embodiment, a system for dynamic evaluation and visualization of user engagement comprises an engagement module including computing hardware of at least one processor and memory operably coupled to the at least one processor, and instructions that, when executed on the engagement module, cause the engagement module to implement a monitoring engine configured to receive behavior data associated with a period of activity of a user, an assessment engine configured to generate an assessment based on the period of activity, and determine an engagement level of the user based on the behavior data and the assessment, and a visualization engine configured to generate an avatar based on the engagement level, and generate a transaction on a blockchain, the transaction indicating the user owns the avatar.

In one aspect, a system further includes a reporting engine configured to implement a dedicated API for a networked user, wherein the networked user modifies the blockchain with a new transaction, the new transaction modifying the avatar.

In one aspect, the transaction on the blockchain includes an engagement profile characterizing the learning ability of the user.

In one aspect, the assessment engine is further configured to determine the engagement level of the user based on a comparison of the behavior data to known behavior data.

In one aspect, the known behavior data comprises historical data of the user, the known behavior data including a dataset that grows over time as the user interacts with the engagement module.

In one aspect, the monitoring engine is further configured to implement a machine learning model to extract the behavior data using a neural network.

In one aspect, a system further includes a digital wallet, wherein the avatar is minted as a non-fungible token in the digital wallet.

In one aspect, the assessment engine is further configured to implement a machine learning model with a feedback component to generate revised behavior data.

In an embodiment, a method for dynamic evaluation and visualization of user engagement using a blockchain comprises receiving behavior data associated with a period of activity of a user, generating an assessment based on the period of activity, determining an engagement level of the user based on the behavior data and the assessment, generating an avatar based on the engagement level, and generating a transaction on the blockchain, the transaction indicating the user owns the avatar.

In an embodiment, a data structure stored on a blockchain includes a learning engagement profile personalized to a user, the learning engagement profile characterizing a learning ability of a user, an avatar including a plurality of design features based on the learning engagement profile, wherein the learning engagement profile and the avatar are updated on the blockchain based on a user interacting with a digital environment and a physical environment.

The above summary is not intended to describe each illustrated embodiment or every implementation of the subject matter hereof. The figures and the detailed description that follow more particularly exemplify various embodiments.

### BRIEF DESCRIPTION OF THE DRAWINGS

Subject matter hereof may be more completely understood in consideration of the following detailed description of various embodiments in connection with the accompanying figures, in which:
FIG. 1 is a block diagram of a system for determining and visualizing student engagement levels, according to an embodiment.
FIG. 2 is a block diagram of blockchain objects and a digital wallet in a system for determining and visualizing student engagement levels, according to an embodiment.
FIG. 3 is a flowchart of a method for implementing a learning profile, according to an embodiment.
FIG. 4 is a flowchart of a method of determining and visualizing student engagement levels, according to an embodiment.

While various embodiments are amenable to various modifications and alternative forms, specifics thereof have been shown by way of example in the drawings and will be described in detail. It should be understood, however, that the intention is not to limit the claimed inventions to the particular embodiments described. On the contrary, the intention is to cover all modifications, equivalents, and alternatives falling within the spirit and scope of the subject matter as defined by the claims.

### DETAILED DESCRIPTION OF THE DRAWINGS

Embodiments of the present disclosure present systems and methods for providing ownership of data using blockchains. Some implementations allow for the provisioning and execution of distributed storage workflows based on that data, for example, to improve security and efficiency. By selectively implementing storage operations between a learning platform and a blockchain, embodiments reduce overhead associated with data growth, increase interoperability (e.g., by counteracting blockchain siloing), and increase usability.

For context and clarity, the present disclosure contemplates use of the technical solutions described herein for measuring and increasing engagement of a user (e.g., student) by creating and dynamically updating a personalized learning environment for the user. These descriptions are intended to serve as an illustrative use case where the underlying technical advantages can be applied to great benefit. It should be appreciated that the provided embodiments for addressing user needs and preferences and should not be considered limiting as to the underlying technical improvements as described herein.

In traditional classrooms education is often not personalized to student needs. Typically, any number of students are taught with the same instructional content at the same pace. This one-size-fits-all approach leads to some students who learn at a faster pace having to wait before being able to move on to the next topic or unit in the course. Similarly, students who learn at a slower pace may be forced to move through the instructional content at a pace that exceeds their capability and, as a result, these students may not be able to devote sufficient time to gaining a mastery of the instructional content. This type of instruction fails to account for student preferences and differences, such as variances in learning styles and areas of interest and is not well-suited to the diverse and individualized needs of learners in today's world.

Embodiments of the present disclosure present overcome these limitations by dynamically allocating storage and retrieval of student data between distributed devices and databases, allowing students to own their education. In embodiments, student data is used to generate a visualization of the educational experience of a student, which facilitates self-reflection and investment. Accordingly, embodiments provide for a dynamic, scalable approach to measuring engagement around learning without the conventional sacrifice of personalization.

In an embodiment, a learning platform is configured to generate a learning profile for a (e.g., each) student. A learning profile identifies trait(s), an engagement profile, and an avatar associated with a student. In an embodiment, the learning platform is configured to store learning profiles in a blockchain.

In an embodiment, learning profiles include student traits. Student traits can include, for example, one or more of learning style, interests, self-regulation, motivation, resilience, curiosity, social skills, cognitive abilities, and prior knowledge. In embodiments, individual student traits can be quantitative or qualitative.

Student traits can include a learning style. Students can have diverse learning styles, including visual, auditory, kinesthetic, and tactile preferences. Understanding and accommodating these preferences can enhance comprehension and retention. In an embodiment, learning style can comprise a relative ranking of learning styles.

Student traits can include an indication of the memory of a student. For example, student traits can include a memory score or metric reflective of memory capacity and retention abilities. By associating a learning profile with a memory score (e.g., the ability of a student to memorize or recall material), an appropriate engagement framework can be provided. For example, techniques such as mnemonic devices and spaced repetition can aid memory retention.

Student traits can include motivation factors for a student. Motivation factors can include intrinsic motivation factors, such as interests, and extrinsic motivation factors, such as external rewards that are preferred or effective for the student. By correlating a learning profile with motivation factors, engagement frameworks maximize student interaction with educational material.

Student traits can include an indication of the self-regulation capabilities of a student. Students with strong self-regulation skills can manage their behavior, emotions, and cognitive processes effectively. These skills can include goal setting and time management.

Student traits can include an indication of the resilience of a student. Resilience enables students to cope with setbacks, persevere through challenges, and maintain a positive attitude toward learning. By incorporating resilience measurements in learning profiles, engagement frameworks can better predict when to dynamically change lessons plans or tasks (e.g., to push the student to learn until a point that is determinantal to their engagement).

Student traits can include prior knowledge. Prior knowledge influences how students interpret new information and make connections to existing concepts. Activating prior knowledge and addressing misconceptions during educational lessons are essential for effective learning.

Student traits can include an indication of the cognitive ability of a student. Individual differences in cognitive abilities, such as problem-solving skills, critical thinking, and spatial reasoning, impact learning performance across various subjects and tasks. By incorporating indications of cognitive ability into learning profiles, engagement frameworks can better pace content and determine appropriate difficulty.

Student traits can include an indication of the social skills of a student. Social skills, including communication, collaboration, and empathy, influence peer interactions, group work, and classroom dynamics.

In an embodiment, learning profiles include engagement profiles. Engagement profiles include indications of behaviors, or lack thereof, that may indicate levels of engagement for a particular user and environment. For example, engagement profiles can include behavior metrics and engagement challenges, such as pitfalls that stand in the way of a student's learning. In an embodiment, engagement profiles can be used to determine an engagement level of the student and/or to develop a plan (e.g., engagement framework) that will move the user to a higher level of engagement. For example, the engagement profile of a student may include an indication of the attention span of the student. The ability to sustain attention affects how well students engage with instructional materials and stay focused during lessons. For example, short attention spans may require varied instructional strategies to maintain engagement.

In an embodiment, learning profiles include avatars. Avatars provide a student with a visualization of their student traits and engagement profile. By recognizing and addressing student traits and engagement, students can better comprehend individual strengths and weaknesses to inform their approach to education, ultimately fostering academic success and lifelong learning skills.

In an embodiment, the learning profile can be based on a set of standard profiles that are associated with respective engagement frameworks and traits. Standard profiles are foundational learning profiles comprising representative sets of student traits and engagement profiles supported by the learning platform. Standard profiles can provide a roadmap for implementing engagement frameworks via the learning platform on a device of the student. For example, when generating a learning profile, the learning platform can determine an initial engagement framework based on mappings of chosen learning elements (e.g., traits and engagement profile attributes) to those of standard profiles. For example, mappings between traits and/or engagement profiles to predefined engagement frameworks can be used. In examples, the standard profiles can be sent from the learning platform to a computing device to reduce complexity of implementing a custom learning profile at a device with intermittent (e.g., unreliable) network connectivity or limited communication capabilities (e.g., bandwidth). In such examples, the learning profile can be generated and implemented with reduced processing overhead and without communication beyond the initial message from the learning platform.

In some implementations, standard profiles can be automatically refined by a global ML model and distributed to participating devices. The participating devices can provide abstracted feedback to the global model without sharing raw data with a central server, allowing for enhanced security. In embodiments, feedback to the global model can be provided periodically, for example, to reduce communications between the participating devices and the learning platform.

In an embodiment, the learning platform can serve as a centralized identity provider for the learning profile of a student, including learning traits of the student. For example, the learning platform can be configured to provide standard profiles that can act as a basis for a personalized learning profile based on user input. The learning platform can store the personalized learning profile and selectively grant access to service provider(s), such as third-party websites, cloud services, or online platforms. The learning platform can authenticate students and authorize access to their resources, for example, by administrators (e.g., parents, teachers) and third-party developers. Use of the learning platform as a central identity provider can allow students to access different systems and services without the need for separate credentials for each. Further, along with authentication, the learning platform can support attribute exchange, allowing the student to share specific elements of their learning profile (e.g., engagement profile and traits) with a service provider. Embodiments of the present disclosure provide for abstraction of learning profile data to allow for personalized experiences and access control based on user attributes while mitigating distribution of sensitive student data.

The learning platform configured for centralized identity provision and distributed storage allocation (e.g., as described herein) can reduce an attack surface by consolidating authentication and access control functions within a trusted identity provider. This minimizes the exposure of sensitive authentication credentials and reduces the likelihood of security breaches resulting from compromised credentials or vulnerable authentication mechanisms. By centralizing authentication and access control, the risk of password-related vulnerabilities (e.g., phishing and password reuse) is reduced and more granular recovery methods and monitoring can be implemented between storage solutions (e.g., user devices, databases, and blockchains). In an embodiment, use of distributed ML operations in combination with a centralized identity provisioning system allows for strengthened security posture.

In an embodiment, use of the learning platform as an identity solution can scale to accommodate large numbers of users and applications, making it well-suited for enterprise environments and distributed systems. For example, the learning platform can serve as an interface to a blockchain of transactions associated with learning profiles. Incorporation of a blockchain as an auxiliary storage mechanism for the learning platform facilitates self-ownership of data while mitigating throughput and latency impacts. In some implementations, allocation of learning profiles between storage solutions can create an immutable record of learning profile(s) without storage of sensitive data within the blockchain itself. Conventional blockchain approaches are often limited due to the inherent transparency of recorded transactions. In embodiments, the determinations made by learning platform to mediate access to storage solutions represent a technical solution to the deficiencies of prior approaches. These determinations can further allow for continued compliance with the ever-evolving regulatory landscape regarding data privacy and security.

In an embodiment, the learning profile of a student is accessible, via the learning platform, by educators and developers to tailor experiences for students. In some implementations, the learning profile is stored via a database (e.g., a SQL database) and a blockchain. A dedicated API can be provided for a student that is configured to modify the blockchain with new transactions related to their learning profile. In embodiments, teachers can have differentiated, customized access to the dedicated API of their students to facilitate reading, and in some cases writing, to the learning profile without direct access to the blockchain or ownership of the learning profile. In some implementations, the learning platform can implement an API gateway to facilitate seamless interaction between blockchain applications and external databases, ensuring efficient data transfer and synchronization. In some embodiments, use of a dedicated API enables administrator (e.g., teacher, parent) to enact blockchain manipulations. These non-student manipulations to the blockchain can be labeled (e.g., through use of the differentiated API), allowing for accountability and auditing capabilities. Accordingly, the learning platform can complement, rather than replace, student ownership and development of learning profiles.

In an embodiment, an educator interface can be used to provide feedback and tailor adjustments to the learning profiles of their students, for example, to reduce the risk of perpetuating bias or discrimination. For example, an ML algorithm that recommends learning materials based on past performance may reinforce existing inequalities by directing students from marginalized groups toward lower-quality resources. By providing administrators (e.g., educators and parents) with visibility into the learning profiles of students and the blockchain, transparency of the system can be improved without sacrificing student ownership or personalization. Accordingly, each student can more effectively develop skills, based on students reaching higher levels of engagement, such as creativity, critical thinking, and problem-solving, while maintaining ownership of their data.

In an embodiment, collaborative filtering can be applied by the personalized learning environment to learning profiles, to further reduce bias and address a technical problem facing data personalization-the cold start problem. The cold start problem arises when a recommendation system lacks sufficient data about a new user or item to generate accurate recommendations. This problem can occur in traditional software solutions, particularly in ML applications that rely solely on user-provided preferences or item metadata to make refinements. Collaborative filtering can resolve the cold start problem by determining user recommendations based on reactions by similar users, particularly through the incorporation of standard learning profiles. Instead of relying solely on explicit user preferences, collaborative filtering analyzes patterns of user behavior and preferences to identify similarities between users and items. In an embodiment, the integration of collaborative filtering to learning profiles can further leverage the behavioral monitoring associated with implementation of engagement profiles without burdening participating devices. For example, collaborative filtering can be applied by the learning platform based on periodic, abstracted feedback from participating devices. The learning platform can update standard profiles and associations between existing learning profiles.

In an embodiment, behavior monitoring, and engagement profiles can be leveraged independently from student traits and sensitive data. By selectively diversifying storage (e.g., siloing) student data, embodiments of the learning platform can provide hierarchical access to sensitive data. For example, data storage associated with a learning platform can be structured hierarchically, with different levels of authority or responsibility assigned to individuals or groups. This hierarchy may be based on factors such as user roles (e.g., student, educator, parent) and classifications (e.g., students of a particular grade level). Hierarchical access control allows for granular control over data access, for example, allowing educational administrators to define different levels of access privileges at each level of the hierarchy. For example, within a personalized learning environment, a high school student may have access to (e.g., all) data associated with their user profile while younger students may only have access to data relevant to their specific responsibilities, such as homework tasks. To ensure compliance and security, access to sensitive data can be audited and monitored. Audit logs record access events, including who accessed which data and when, allowing administrators to track access patterns, detect unauthorized access attempts, and investigate security incidents. In embodiments, blockchain technologies can be leveraged to enhance auditing and reporting capabilities.

As students progress through their educational journey in both digital and physical environments, the learning platform can continually update the learning profiles of students and associated avatars (e.g., visual representations). For example, traits and engagement profiles stored on the blockchain can prompt an avatar update for each new transaction. For example, the personal learning environment can employ incremental learning techniques to update predictive models and recommendation algorithms periodically (e.g., instead of continuous reporting as new data becomes available). This can reduce computational overhead on local devices, especially when compared to retraining models from scratch, and enable adaptive learning experiences.

Embodiments of the present disclosure provide for a more effective and personalized learning platform that incorporates unique allocation of ML workflows and a blockchain to overcome technical issues of conventional personalization systems and environments.

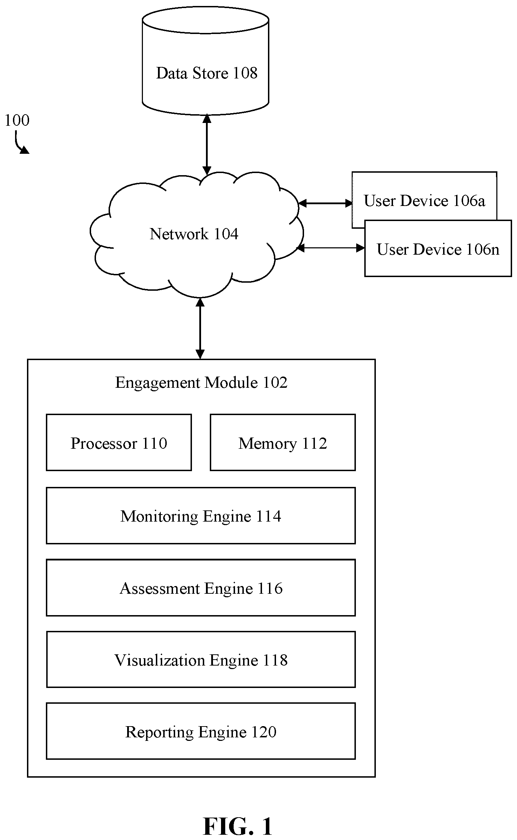

Referring to FIG. 1, a block diagram of system 100 for providing a dynamic learning platform is depicted, according to an embodiment. System 100 is configured to generate and maintain learning profiles for students and generally comprises engagement module 102, network 104, user device(s) 106, and data store 108.

In embodiments, engagement module 102 is configured to generate and maintain personalized learning profiles. Engagement module 102 generally comprises processor 110, memory 112, and one or more engines, such as monitoring engine 114, assessment engine 116, visualization engine 118, and reporting engine 120.

Engagement module 102 generally generates and updates learning profiles, including engagement profiles and traits, of users by providing a set of standard profiles (e.g., traits and engagement profiles) to act as a baseline for learning profiles, creating a learning profile based on a selection of elements from the set of standard profiles and user input, implementing an engagement framework associated with the selected standard profile elements, identifying behaviors, or lack thereof, that may indicate levels of engagement for a particular user and environment, connecting the identified behaviors to a framework for explaining the user's current engagement, developing a plan that will move the user to a higher level of engagement, and presenting a visual representation of user specific engagement and learning preferences. In embodiments, the functionality of engagement module 102 is accomplished through monitoring engine 114, assessment engine 116, reporting engine 120, and visualization engine 118, as will be described.

Embodiments described herein include various engines, each of which is constructed, programmed, configured, or otherwise adapted, to autonomously carry out a function or set of functions. The term engine as used herein is defined as a real-world device, component, or arrangement of components implemented using hardware, such as by an application specific integrated circuit (ASIC) or field-programmable gate array (FPGA), for example, or as a combination of hardware and software, such as by a microprocessor system and a set of program instructions that adapt the engine to implement the particular functionality, which (while being executed) transform the microprocessor system into a special-purpose device. An engine can also be implemented as a combination of the two, with certain functions facilitated by hardware alone, and other functions facilitated by a combination of hardware and software. In certain implementations, at least a portion, and in some cases, all, of an engine can be executed on the processor(s) of one or more computing platforms that are made up of hardware (e.g., one or more processors, data storage devices such as memory or drive storage, input/output facilities such as network interface devices, video devices, keyboard, mouse or touchscreen devices, etc.) that execute an operating system, system programs, and application programs, while also implementing the engine using multitasking, multithreading, distributed (e.g., cluster, peer-peer, cloud, etc.) processing where appropriate, or other such techniques.

An (e.g., each) engine can be realized in a variety of physically realizable configurations and should generally not be limited to any particular implementation exemplified herein, unless such limitations are expressly identified. In addition, an engine can itself be composed of sub-engines, each of which can be regarded as an engine in its own right. Moreover, in the embodiments described herein, each of monitoring engine 114, assessment engine 116, visualization engine 118, and reporting engine 120 correspond to a defined autonomous functionality; however, it should be understood that in other contemplated embodiments, each functionality can be distributed to more than one engine. Likewise, in other contemplated embodiments, multiple defined functionalities can be implemented by a single engine that performs those multiple functions, possibly alongside other functions, or distributed differently among a set of engines than specifically illustrated in the examples herein.

Engagement module 102 can be implemented irrespective of the number or type of engines. In embodiments, one or more of monitoring engine 114, assessment engine 116, visualization engine 118, and reporting engine 120 can operate on distinct servers.

Processor 110 can be any programmable device that accepts digital data as input, is configured to process the input according to instructions or algorithms and provides results as outputs. In an embodiment, processor 110 can be a central processing unit (CPU) or a microcontroller or microprocessor configured to carry out the instructions of a computer program. Processor 110 is therefore configured to perform at least basic arithmetical, logical, and input/output operations.

Memory 112 can comprise volatile or non-volatile memory as required by the coupled processor 110 to not only provide space to execute the instructions or algorithms, but to provide the space to store the instructions themselves. In embodiments, volatile memory can include random access memory (RAM), dynamic random access memory (DRAM), or static random access memory (SRAM), for example. In embodiments, non-volatile memory can include read-only memory, flash memory, ferroelectric RAM, hard disk, or imaging disc storage, for example. The foregoing lists in no way limit the type of memory that can be used, as these embodiments are given only by way of example and are not intended to limit the scope of the present disclosure.

Monitoring engine 114 is configured to identify behaviors that may indicate levels of engagement for a particular user by tracking and interpreting user activity across user devices 106. Determinations of monitoring engine 114 from engagement data can be based on, for example, an engagement profile associated with the learning profile.

For example, students who want their activity to be reflected in their individualized engagement profile can toggle activity monitoring. The ability to opt in or out of activity monitoring can be accomplished through an engagement widget available through a web-browser (e.g., an extension) or an application on user device 106, according to embodiments. In embodiments, activity can be monitored automatically for programs that incorporate one or more elements of the dynamic learning environment through use of an application programming interface (API). In classroom environments, teachers can allow activity monitoring for their students during instruction periods such that additional (and third party) input can be provided to monitoring engine 114.

In an embodiment, monitoring engine 114 is configured to receive abstracted data from user devices 106. Abstracted data refers to data that has been transformed or simplified to remove specific details, such as user-identifying data. The receipt of abstracted data can improve the efficiency of monitoring engine 114 by making the data more generalized and easier to interpret while still retaining its essential characteristics or properties. In an embodiment, the abstraction process implemented by user device 106 involves distilling complex data into higher-level representations, allowing monitoring engine 114 to focus on key insights or patterns across user profiles without handling large volumes of raw data. Moreover, the abstraction of data by user device 106 can reduce network traffic as complex datasets are simplified before communication.

In embodiments, monitoring engine 114 can implement ML such that monitoring is improved. For example, monitoring engine 114 can implement a neural network to learn from previous mistakes in monitoring. In embodiments, limited human assistance is required because the neural network can learn and model the relationships between input and output data, as described herein.

Monitoring engine 114 interprets student interactions with content. Interaction metrics can include one or more of detected behaviors, time between activity, and type of activity registered. Where the student is expected to provide a particular action, such as a short form answer to a question, characteristics (e.g., time to respond, length of response, prevalence of grammatical errors) of any provided input can be analyzed. In embodiments, monitoring engine 114 can be communicatively coupled to user device 106 to monitor a student's environment, such as through audio or video analysis.

Disengaged or avoiding behaviors (i.e., level 1) can include one or more of a student being absent from a class or activity, acting out, inquiring about something off topic, and activity on non-classroom applications or websites. Confused behaviors (i.e., level 2) can include one or more of meltdowns, not working on or giving up on an assigned task, and chatting or messaging with peers. Distracted behaviors (i.e., level 3) can include one or more of incomplete work, rushing to complete a task, fidgeting, and not maintaining eye contact. Following instructions behaviors (i.e., level 4) can include one or more of working on a task quietly, maintaining a calm demeanor, completing an assigned task, and making connections with learning material. Incentivized or extrinsic motivation behaviors (i.e., level 5) can include one or more of consistently asking about an incentive, becoming distracted by an incentive, and reminding an educator of an incentive. Inspired behaviors (i.e., level 6) can include one or more of being excited about working towards goals, spending free time on a task or topic, asking good clarifying questions about a task, and completing tasks without requiring additional oversight or guidance. Deeply passionate behaviors (i.e., level 7) can include one or more of talking about or sharing learning material with others, actively participating in an activity, advocating for a topic or task, and taking on a leadership role.

While described as related to a particular level of engagement, it is important to note that particular detected behaviors can be categorized differently depending on the task, student characteristics, duration of detected behavior, severity of detected behavior, and learning environment factors. Learning environment factors can include physical or technical distractions such as physical discomfort, technology issues, loud noises, other student behavior, and significant home life events. When a detected behavior is detected or received, contextual data surrounding the instance of the detected behavior can be recorded. For example, the time of day and type of activity can be recorded and used by monitoring engine 114 to update a student's learning profile. Accordingly, monitoring engine 114 can process multiple types of data simultaneously to update a student's learning profile. More particularly, monitoring engine 114 can capture primary data and secondary data such that the secondary data impacts the primary data value.

In embodiments, user device 106 is configured to aggregate (e.g., average) interaction metrics over time. For example, rather than communicating interaction metrics in real-time, user device 106 can provide a periodic report to monitoring engine 114. In embodiments, user device 106 can generalize data by abstracting specific instances or examples into more generalized concepts or categories. For instance, grouping similar responses into response categories or summarizing individual student assessments into broader educational progress.

In an embodiment, monitoring engine 114 can recognize the capabilities of user device 106 to proactively determine a reporting type of user device 106. The reporting type can be one of remote monitoring, continuous reporting, or batched reporting. For example, monitoring engine 114 can request specifications of user device 106 to determine an appropriate reporting type. In an embodiment, the reporting type of a group can be specified by an administrator. For example, an educational institution can specify that all or particular user devices can only provide batched reporting.

In an embodiment, remote monitoring comprises monitoring engine 114 processes an ongoing data feed (e.g., a video bitstream) from user device 106, such that monitoring engine 114 tracks the current engagement state of a student. Remote monitoring may allocate a higher burden on networking capabilities while reducing local processing of user behavior on user device 106. Remote monitoring may raise data security concerns as the contents of the data stream sent by user device 106 can be unfiltered. Remote monitoring can be used, for example, when user device 106 is using a virtual computing environment or cloud service that has sufficient networking capabilities but presents a temporary environment (e.g., where storage of monitored data is unsupported between sessions) or lacks storage capabilities (e.g., fails to meet set security standards). In an embodiment, remote monitoring capabilities can be selectively engaged for particular activities, such as assessments.

In an embodiment, continuous reporting comprises user device 106 reporting detected behaviors to monitoring engine 114 frequently (e.g., in real-time). Continuous reporting can be advantageous as abstraction and/or generalization can be applied to data prior to reporting.

In an embodiment, batched reporting comprises user device 106 periodically providing detected behaviors to monitoring engine 114, such that the reporting is delayed. For example, batched reporting can be used when metrics are provided by a computing device in aggregate.

In embodiments, monitoring engine 114 is configured to receive educator and/or parent observations. Observing a learner's behavior and participation in a learning activity can provide additional insight into their level of engagement. For example, a teacher may observe a student's participation in class discussions, or their ability to stay focused and on task during an independent learning activity. In such embodiments, monitoring engine 114 can prompt users for specific feedback through questions or surveys that are correlated to engagement levels. Observed learner behavior can be saved to the profile to improve knowledge transfer through the student's educational journey. Having engagement and learning style history tracked in a centralized source that is owned by the student improves the student's ability to self-advocate and articulate their own learning needs. Moreover, such aggregation by monitoring engine 114 provides consistency in the student's profile compared to traditional solutions which are typically not integrated in one source.

Educator and parent observations can include in-person behaviors or physical environment actions. Physical environment behaviors can be categorized into levels of engagement similar to detected behaviors by monitoring engine 114. For example, classroom environment behaviors that correspond to a disengaged or avoiding engagement level can include one or more of sharpening pencils, placing head down, drawing or doodling, talking to peers, or attempting to leave the room.

Behaviors that can be observed by monitoring engine 114 can include overall participation behaviors, session participation behaviors, and session-adjacent behaviors.
Overall participation behaviors generally pertain to a student's interaction and progress with one or more programs related to the dynamic learning environment. Overall participation behaviors can include one or more of: a student signed up for the program, but has never attempted to participate in the program; a student signed up for the program, and attempted to participate, but has not yet completed a session; a student has signed up for the program, and completed a session, but never returned for another session; a student has signed up for the program, and completed multiple sessions; and a student has signed up for the program and completed enough sessions to have made measurable learning progress.

Session participation behaviors generally pertain to interaction that is expected during a session (i.e., a lesson or an educational program). Accordingly, session participation behaviors generally are only present when a student is participating in a session. Session participation behaviors can include one or more of: a student "raised their hand" within a session, they want to chime in; a student "accepted" an invitation to actively participate (e.g., come on stage); a student "declined" an invitation to actively participate; a student answered a multiple choice question posed to the group, participating in helping decide what to do; a student answered an open response question, providing an answer or idea in their own words; a student shares an emoji or emoticon during a live, interactive session.

Session-adjacent behaviors generally pertain to student activities preceding, during, or after a session that do not pertain to the session object or session-associated interactions. Session-adjacent behaviors can include one or more of: a student has collaborated with a friend on the platform to build something new within the experience; a student has submitted ratings on the guide/session (given opportunity at the end of each session to rate).

In embodiments, identified behaviors are each associated with one or more levels of engagement that can quickly and accurately profile a learner's style of learning and current engagement. Levels of engagement can include one or more of disengagement, unsystematic engagement, frustrated engagement, structure dependent engagement, self-regulated interest, critical engagement, and literate thinking. Each level of engagement can be seen as a hierarchy with the lowest point being engagement and the highest point being an active participant or creator. Association of behaviors to a student's current level of engagement allows dynamic determination of next steps to drive interaction. Steps taken to improve engagement include one or more of providing the student with a prompt, generating a call to action, providing a reward or incentive, and changing session objectives or difficulty.

A student's level of engagement is dynamic and can change during any interaction with the personalized learning environment. Further, a change in engagement level can be nonsequential. For example, a student with an observed engagement level of self-regulated interest, which in embodiments can be numerically represented as the fifth engagement level of seven, can be moved to an engagement level of disengagement, returned to the first engagement level, based on an observed action of disinterest or absence of action for a prolonged period of time where action is anticipated. Accordingly, in embodiments, a level of engagement can be represented as a vector in vector space. A data point with multiple features can be represented as a vector (e.g. the various levels of engagement over time during interaction with the personalized learning environment). Vectors can be compared against other vectors in vector space using ML algorithms, as is described further herein. In one aspect, monitoring engine 114 is further configured to weight student interactions based on detected levels of engagement. Weighting can include an emphasis on certain behaviors or characteristics for the levels of behaviors further discussed below. In an embodiment, a particular behavior, as determined for a certain level of engagement, can include a relatively higher or lower weighting factor. For example, in level 1 behaviors that show avoiding or idle, certain behaviors can be weighted according to TABLE 1 below The weighting can be utilized for level calculations (e.g. in a sum, average, weighted average, or other suitable calculation).

**TABLE 1**

| **Behavior** | **Weighting** |
|---|---|
| "Not at school" | 1 |
| "Acting out" | 3 |
| "Trying to get the teacher's attention about something off topic" | 3 |
| "Head down" | 2 |

In an embodiment, weighting can include if-then determinations such that certain behaviors are linked together for markers of the engagement levels. For example, if the behavior "distracting other students" (e.g., which can have a certain weight alone) is followed by "asking a lot of random questions" (e.g., which can also have a certain weight alone), the combined if-then weighting can be greater than the weights of the two behaviors alone.

In embodiments, student interactions are associated with each level of engagement such that level 1 behaviors show avoiding or idle, level 2 behaviors show confusion with messaging or receiving instructions, level 3 behaviors are distractions or result from distractions, level 4 behaviors represent following instructions, level 5 behaviors are based on rewards, level 6 behaviors are based on goal setting and inspiration (e.g., manipulating the experience, building something, creating something, collaborating), and level 7 behaviors are focused on being an ambassador and sharing the experience, identifying the experience. Detection of behaviors can be accomplished through sentiment analysis and ambassador programs. In embodiments, some detected behaviors qualifiers can be used to determine the appropriate level of engagement more accurately. For example, if a student user is presented instructions and then does not follow instructions or disconnects from the session the behavior can be weighted differently than a student who had yet to receive instructions. Weighting of student interactions can further be based on a student's impact on the engagement of other students.

In operation, separation of behaviors into specific levels of engagement can improve efficiency of behavior identification. By only observing for certain behaviors that are relevant to the student's current level of engagement, a reduction in processing overhead is realized. More particularly, behaviors can be selectively identified based on the level of engagement. Accordingly, this technique can improve efficiency by reducing the size of data being transmitted and analyzed from each student's device during the monitoring process. This is particularly important when a student may be experiencing an unreliable network connection or when the networking capabilities of a student's device are limited. To further reduce the burden placed on network-connected devices, a student's activity can be saved locally until an upload process is initiated. In some embodiments, student activity can be evaluated locally on the student's device. Such enhancements represent advantages over conventional monitoring techniques that are prohibitive for sustained periods of time, whether for networking or processing capability purposes. Further, reduction of processing overhead and/or system complexity is particularly important in the educational context as some students must rely on computing devices provided by their educational institutions, which can often be antiquated or limited in functionality.

In embodiments, monitoring engine 114 is configured to leverage behavioral insights to overcome shortcomings of traditional educational systems that fail to personalize education to a student needs. In traditional settings, any number of students are taught with the same instructional content at the same pace. This one-size-fits-all approach leads to some students who learn at a faster pace having to wait before being able to move on to the next topic or unit in the course. Similarly, students who learn at a slower pace may be forced to move through the instructional content at a pace that exceeds their capability and, as a result, these students may not be able to devote sufficient time to gaining a mastery of the instructional content. This type of instruction fails to account for student preferences and differences, such as variances in learning styles and areas of interest and is not well-suited to the diverse and individualized needs of learners in today's world. By capturing behavioral data on students during the learning process, insights can be made to facilitate each student learning at pace and with content most effective for their needs and preferences.

With continued reference to FIG. 1, assessment engine 116 is configured to evaluate user comprehension, personal development, and engagement with instructional material through the incorporation of interactive elements. Interactive elements can include surveys, self-assessments, observational time sampling, individual games, collaborative games, interviews with students and teachers, and teamwork exercises. In embodiments, the interactive elements can be presented through a web browser or downloadable application and are used to generate and update individualized avatars for each student as will be described in relation to visualization engine 118.

Assessment engine 116 can ensure that the learning profile for each student dynamically updates as the student develops a better understanding of their learning styles and interest areas. Input received by assessment engine 116 can be associated with one or more levels of engagement. In embodiments, the levels of engagement can be categorized into the pitfalls of learning, productivity, and passion, and purpose. If a student indicates that they are distracted by over-stimulation, this feedback can be associated with a pitfall of learning and later be used to determine appropriate ways to engage the student, should they fall to a low level of engagement. Therefore, as each student continues to interact with engagement module 102 the dataset used as a basis to inform future engagement recommendations and drive learning decisions grows.

In an embodiment, students who indicate similar traits, engagement preferences, and/or learning styles can be associated. Associations between students can be used to group like students for experiences that are most beneficial for their learning style, generate recommended actions to improve engagement, and create a sense of community within the personalized learning environment. Consideration of similar students can lead to enhanced recommendations and learning pathways for newer students who might have a limited assessment dataset. For example, collaborative filtering can be applied to determine standard profiles that can be used to generate new learning profiles.

Self-assessments, including surveys or questionnaires, generated by assessment engine 116 can provide learners with a way to reflect on their own level of engagement and interest in the material being taught. Surveys ask learners to rate their engagement on a scale or provide additional feedback on their engagement or progress. Self-assessment surveys as they currently do exist can be useful for measuring engagement around learning, but they also have some potential limitations. One problem with conventional self-assessment surveys is that people's self-assessments may not always accurately reflect their true level of engagement. For example, someone may overestimate their level of engagement because they want to appear more interested or committed than they actually are. Additionally, self-assessment surveys rely on the individual's own subjective judgment, which can be influenced by factors such as their mood or their personal beliefs about the importance of learning. Embodiments of the present disclosure reduce self-reporting concerns, by combining (e.g., weighting) self-assessments with other methods for measuring engagement, such as observation or feedback from others as tracked by monitoring engine 114. Moreover, embodiments utilize levels of engagement such that the student does not self-assess their own engagement. Rather, user input (e.g. from a student survey) is applied to data for the levels of engagement to create a profile for the student user. Accordingly, a more accurate characterization of the student user is created by utilizing input applied to known data instead of a profile created directly from self-assess data itself.

Assessment engine 116 addresses the inefficiencies of traditional learning environments that are based on indirect or proxy measures of engagement and comprehension, such as attendance or test scores (e.g., which may not accurately reflect a student's true level of engagement). Measures, such as test scores, are static and do not provide real-time or ongoing feedback, which can make it difficult to identify and address engagement and comprehension issues in a timely manner. Finally, many of the existing measures of engagement can often introduce anxiety for students as each periodic measure of progress can have lasting impacts in the student's educational journey. Assessment engine 116, in conjunction with other engines of engagement module 102, can develop assessment measures on a student-by-student basis to reduce student friction with the educational process and allow for better self-understanding.

Visualization engine 118 can provide visualized feedback for responses to promote honest reporting as the student receives personalized incentives that reflect their indicated engagement preferences or learning style, contrasting traditional classroom self-assessment that provides no apparent benefit for truthfully representing disengagement.

Self-assessments generated by assessment engine 116 coincide with professional development and session plans for both the students and educators to gauge engagement levels through questions. In embodiments each question can be associated with the levels of engagement on a scale of 1-7 such that each provided response correlates to a unique level of engagement. So rather than a student having to determine how "engaged" they are on a sliding scale, the student simply answers a question. Each provided response coincides with a level of engagement based on the behaviors exhibited and not a number the student associates with their level of engagement. This nuanced approach to the self-assessment process allows for the students to better communicate learning roadblocks or distractions and gives teachers real-time feedback on how to then support that student based on the diagnostics.

An example self-assessment survey could be presented as: Which of the following statements best describes how you felt about this lesson? Select the letter.
A. This was not for me, no thank you
B. This was too confusing
C. I can't stay focused
D. You asked, and it's easy, so I did it
E. I only did it because I thought I could get something
F. Count me in, this can make a difference
G. This was meant for me, I loved everything about it

In this example, A-G corresponds to engagement levels 1-7. Presenting the information in this manner simplifies the process for the student and can elicit a more honest answer.
Engagement can also be measured through a student's performance on assessments or evaluations provided by assessment engine 116. For example, a student's grades on exams or quizzes can provide insight into their level of engagement with the material being taught. Embodiments of the present disclosure improve upon traditional, standardized evaluation methods by tracking individualized progress of material. Comparing each student's progress to their own demonstrated abilities can provide insights into challenging areas or learning style preferences that otherwise might be overlooked. For example, a student who traditionally excels in science may struggle on a particular assessment but still score highly compared to their peers. Assessment engine 116 can compare their performance to their historical dataset in order to highlight the material or method of instruction as potential learning hazards going forward. This approach makes engagement and learning evaluation more personalized, sustainable, and scalable as it depends on the student's personal learning preferences, learning styles, and ongoing learning abilities rather than comparative performance to other students or the specific goals of any particular learning activity.

Lower levels of engagement, or basic productivity, can be measured, but the higher levels of engagement that showcase a student's deep connection to the material and application of that knowledge tends to be limited in how it is assessed through traditional quizzes and exams. Embodiments of the present disclosure pull data and insights when the child is learning outside the classroom and inside the classroom to assess the student's engagement even when at higher levels. Tracking digital insights of a student's activity can not only personalize the learning experience in the classroom but also validate the learning of the student.

Visualization engine 118 is configured to generate an individualized avatar for each student. An (e.g., each) avatar can serve as a unique identifier that can be used as a passport for accessing learning programs that incorporate the personalized learning environment.

As a student progresses through their learning journey, they can earn various accessories or personalization options for their avatar that reflects their engagement preferences, learning styles, and areas of interest. For example, if a student is distracted by over-stimulation, they could represent this as a pitfall of their learning, which can be translated to sound-proof headphones on an avatar. That student can then see other children with sound-proof headphones and feel a part of a community of similar learners. Further, if the student demonstrates effort to overcome this pitfall of learning visualization engine 118 can generate additional styles of headphones to award this progress. In another example, if a child is passionate about learning about creativity and chooses that as an interest area, visualization engine 118 can generate a visual element for the student's avatar, such as a paintbrush for the student's avatar to hold.

In an embodiment, avatar design features are based on user interactions with one or more environments over time as monitored and evaluated by engagement module 102, including monitoring engine 114 and assessment engine 116. In an embodiment, design features are summarized as follows in TABLE 2 below For example, design features can include differently shaped appearance elements, differently colored appearance elements, and the addition or removal of certain appearance elements altogether.

**TABLE 2**

| **Trait** | **# of Options / Traits** | **Assessment Topic** | **Level** |
|---|---|---|---|
| Ear Color | 2 | THE TOOLS THAT HELP ME DO MY BEST WORK ARE | 1 |
| Head Color | 3 | IN ORDER TO BEGIN TO SIT DOWN AND WORK (Physical-internal) | 1 |
| Nose Color | 3 | I AM MORE COMFORTABLE WORKING ON SOMETHING WHEN | 1 |
| Body Color | 3 | THE SPACE I LIKE TO WORK BEST IS | 1 |
| Mouth Color | 3 | IF I'M CONFUSED, I LIKE IT WHEN | 2 |
| Ears | 4 | INSTRUCTIONS ARE EASIEST TO FOLLOW WHEN | 2 |
| Buttons | 2 | IN ORDER TO KEEP FOCUSING ON MY WORK (ENVIRONMENT) | 3 |
| Body Shape | 2 | WHAT REALLY DISTRACTS ME IS | 3 |
| Background Color | 4 | IN ORDER TO KEEP FOCUSING ON MY WORK (PHYSICAL/MOVEMENT) | 3 |
| Eyes | 4 | WHEN I CAN'T FOCUS ON MY WORK I NEED: | 3 |
| Headgear Color | 2 | WHEN LEARNING HOW TO DO A PROBLEM (REITERATE TEACHER VS PEER) | 4 |
| Nose | 4 | WHEN I WANT TO OFFER AN ANSWER IN CLASS (GROUP OR INDIVIDUAL) | 4 |
| Mouth | 5 | I WOULD BE MOST EXCITED TO DO MY BEST WORK IF I KNEW I WOULD GET | 5 |
| Collar Color | 3 | INDEPENDENCE | 6 |
| Headgear | 3 | I AM MOST INSPIRED TO LEARN WHEN | 6 |
| Stickers | 5 | I LOVE LEARNING THE MOST WHEN | 7 |
| Head Shape | 5 | I FEEL BEST WHEN I AM RECOGNIZED FOR: | 7 |

Visualizing engagement insights for a student in the form of an avatar improves engagement for several reasons. First, using an avatar to represent a student's engagement data can make it more engaging and interactive. By providing a visual representation of the data, students are more likely to pay attention to and engage with the information. Additionally, having ownership of an avatar that the student can customize and personalize can give the student a greater sense of ownership and control over their own learning. This can make students more invested in the learning process, which can increase their motivation and engagement. Additionally, ownership of an avatar makes the data more tangible, which can help the student see the value of the data collected and how the data relates to the student's own learning strengths.

In an embodiment, avatar generation and representation can be dynamically stored as part of the learning profile in a blockchain. As will be described in greater detail, new transactions in the blockchain can be used to update the avatar based on historical data associated with the learning profile such that avatar updates are based on previous avatar visual appearance elements representative of earlier educational progress (e.g., avatars become more unique over time).

Reporting engine 120 is configured to provide reporting of student progress and insights to interested parties. In embodiments, reporting engine 120 provides an interactive dashboard for students and read-only insights to educators. Educators can be provided with groupings and associations between their students to better understand classroom trends and preferences.
In embodiments, reporting engine 120 can comprise a dedicated API to provide a simple and seamless way for any institution or software developer to incorporate one or more elements of each student's dynamic learning environment. One such element that can be leveraged in digital environments is the student's learning passport as generated by reporting engine 120. A learning passport represents a summary or report of a student's progress to date and can include any insights determined from interacting with engagement module 102. In some implementations, the learning passport can include the avatar associated with the learning profile, which can be representative of determined insights. For example, the dedicated API can allow third-party developers to seamlessly incorporate a student's avatar into new experiences, games, and instructional content thereby associating new products with an engagement framework familiar to students. This ease of third-party access contributes to the development of the student's personalized learning profile by facilitating collection of learning insights even when outside of the physical classroom. Accordingly, the use of an API can promote reporting of ongoing insights of trackable behaviors in a digital environment that can be mapped into any data suite for thousands of game players to be scaled with AI.

For example, reporting engine 120 can be accessed across two or more networks, such as by a first software application on a first network and a second software application on a second network. The first software application can be instructional content such that the student user interacts with in a digital classroom. The second software application can be a game the student user plays at home after school. Both experiences can be integrated into the student's learning passport by engagement module 102.

Dedicated API access to engagement module 102 can be controlled by one or more of the students, the students' guardians, or the educational institution in which the student is enrolled. In embodiments control and access of dedicated API permissions is independent from ownership of the data that is controlled by the student.

Reporting engine 120 can also be used to generate and maintain dashboards for each student. Dashboards serve as a centralized source of information for each student. In embodiments, dashboards can be used to explain the engagement framework by connecting identified behaviors to the level of engagement for each user, control permissions and access levels granted to third-parties and educational institutions, customize of the student's avatar, self-report results from lessons and tasks, and track learning and skills progressions. In embodiments, guardians and educators can access a student's dashboard to report on continued learnings and monitor students' skills and learning progressions.

In embodiments, reporting engine 120 is a decentralized reporting mechanism configured to measure engagement across each student's learning journey, allowing students to apply insights from their personalized engagement profile in other settings.

In embodiments, network 104 can be in communication with a server, such as a cloud-based server, that can include a memory and at least one data processor. In such embodiments the server can remotely process one or more computing tasks of engagement module 102.

User device 106 generally comprises processing and memory capabilities and can establish a wireless or wired connection with network 104 or otherwise communicate to engagement module 102, such as by Bluetooth or ad hoc Wi-Fi. Examples of user device 106 include smartphones, tablets, laptop computers, wearable devices, other consumer electronic devices or user equipment (UE), and the like. The term user device is used herein throughout for convenience but is not limiting with respect to the actual features, characteristics, or composition of the or any device that could embody user device 106. In embodiments, user device 106 can run an instance of a user interface designed to facilitate user interaction with one or more features of engagement module 102.

The user interface can include data fields configured to receive user inputs and provide user outputs regarding configuration and status of system 100 and/or engagement module 102. For example, the user interface can present a student's dashboard as generated by reporting engine 120. In some embodiments, the user interface can comprise a mobile application, web-based application, or any other executable application framework. In such embodiments, the user interface can reside on, be presented on, or be accessed by any computing devices capable of communicating with the various components of system 100 and/or engagement module 102.
User device 106 is configured to provide two-way data communication with network 104 via a wired or wireless connection. User device 106 can, via network 104, access stored data from at least one data store 108.

Data store 108 can be a general-purpose database management storage system (DBMS) or relational DBMS as implemented by, for example, Oracle, IBM DB2, Microsoft SQL Server, PostgreSQL, MySQL, SQLite, Linux, or Unix solutions. Data store 108 can store one or more data sets associated with user device 106. In embodiments, data store 108 can be native to engagement module 102 such that no connection to network 104 is necessary.

Ownership of data is assigned to a (e.g., each) student to allow the student to track and measure their personal engagement across all aspects of their learning journey. By leveraging their data students can use the personalized engagement profile to self-advocate for their own needs, preferences, and interests.

When a student has ownership over the data related to their learning, it can have a positive impact on their engagement. This is because owning data can give students a greater sense of agency and control over their own learning. For example, if a student is able to track their progress on a particular assignment or test, the student can see their progress and status in real-time, which can help the student stay motivated and engaged. Additionally, a (e.g., each) student owning data associated with the student can help the student identify their own strengths and weaknesses, which can allow the student to tailor their learning approach to better suit their needs. This can also help students develop a growth mindset, which is the belief that their abilities can be developed through effort and practice.

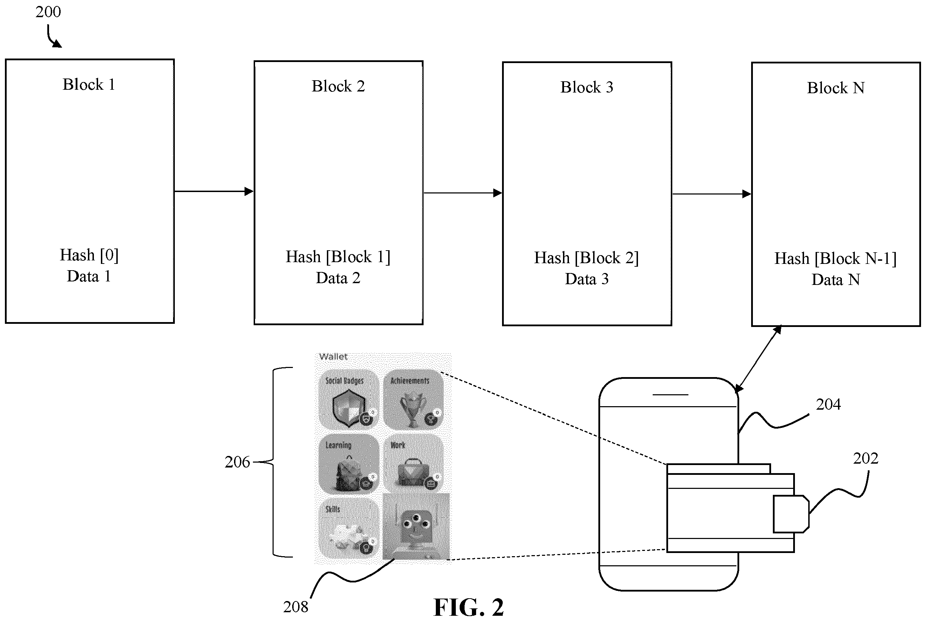

In embodiments, data store 108 is communicatively connected to the network 104, or communicable by the network 104, and is a distributed sequential transactional database such as a blockchain database. For example, and referring further to FIG. 2, a block diagram of blockchain objects 200 and a digital wallet 202 on a computing device 204 are depicted, according to an embodiment. In other embodiments, as will be described, data store 108 can comprise a distributed ledger. As described herein any of the engines of engagement module 102 such as monitoring engine 114, assessment engine 116, visualization engine 118, and/or reporting engine 120 can communicate with data store 108 via network 104.

For convenience, such a distributed sequential transactional database is herein referred to as a blockchain though it will be appreciated that other suitable databases, data structures or mechanisms possessing the characteristics essential for embodiments of the present disclosure could alternatively be used. A blockchain is a distributed chain of block data structures accessed by a network of nodes, referred to here as a miner network. In an embodiment, a plurality of blockchain objects 200 are a distributed chain of block data structures configured to store transaction data as described herein. A (e.g., each) block in the blockchain includes a plurality of record data structures known as transactions, with a (e.g., each) transaction referring to or relating to a prior transaction. For example, a blockchain includes a Merkle of hash or digest values for transactions included in the block to arrive at a hash value for the block, which is itself combined with a hash value for a preceding block to generate a chain of blocks (e.g., blockchain). As depicted in FIG. 2, Hash [0] in Block 1, Hash [Block 1] in Block 2, Hash[Block 2] in Block 3, continuing to Hash[Block N-1] in Block N.

A new block of transactions is added to the blockchain by miner software, hardware, firmware or combination systems in a miner network. The miners are communicatively connected to sources of transactions and access or copy the blockchain. A miner undertakes validation of the substantive content of a transaction and adds a block of new transactions to the blockchain when a challenge is satisfied as a proof of work, typically such challenge involving a combination hash or digest for a prospective new block and a preceding block in the blockchain and some challenge criterion.

In embodiments, the way the blockchain and network of miners operate promotes the adoption of verifiably valid transactions as new blocks added to a blockchain in a manner that is persistent within the blockchain. Transactions added erroneously or maliciously are not verifiable by other miners in the network and their persistence in the blockchain is undermined. This attribute of blockchains is leveraged by embodiments of the present invention to promote secure and individualized ownership of each student's data.

In embodiments, "tokens" can be used to represent and transfer assets via the blockchain. A token serves as an identifier that allows the real-world item, that is, an asset, to be referenced from the blockchain. Similar to physical assets, the tokens that represent physical assets may have many properties, one of which is fungibility or non-fungibility. In economics, fungibility refers to the equivalence or interchangeability of each unit of a commodity with other units of the same commodity. Fungible tokens (FTs) are tokens that can be exchanged for any other token with the same value.

Fungible tokens are uniform, that is, FTs of the same type are identical in specification (e.g., a FT is identical to each other FT of the same type). FTs are divisible into smaller amounts. Similar to currency, where bills can be divided into coins of an equivalent value, FTs are divisible. As such, a fraction of an FT can be transferred between users. Nonfungible tokens (NFTs), however, cannot be replaced with other tokens of the same type. NFTs represent nonfungible assets. Nonfungible assets have unique information or attributes. A (e.g., each) NFT is unique and differs from other tokens of the same class. For example, while plane tickets from and to a same destination may look the same, each plane ticket has a different passenger name, seat number, etc., and, therefore, is unique. In contrast to FTs, NFTs cannot be divided, the elementary unit of the NFT is the token itself.

In the context of the present disclosure, a student's personalized learning profile, including their avatar, can be stored, and treated as NFTs owned by the student and stored in a blockchain (e.g., data store 108 in FIG. 1, and further, Data1... Data N in FIG. 2). In some embodiments, ownership of the NFTs can be automatically transferred from a guardian to the student upon reaching a certain age or educational level. Blockchain technology in conjunction with personalized learning profiles being minted as NFTs can function as verifiable proofs of authenticity and ownership within the blockchain network.

Referring again to FIG. 2, a digital wallet 202 can include an engine or service allowing the student user to make transactions electronically with blockchain 200. As illustrated, digital wallet 202 can be implemented on computing device 204 such as a phone, laptop, or other compatible electronic device (or user device 106 as depicted in FIG. 1).

One or more portions of the personalized learning profile are visualized in the digital wallet at 206. In embodiments, a student's avatar can be automatically minted as an NFT 208, providing the learner with a unique identifier that can be used when accessing learning systems. The avatars are displayed on the student's profile in their digital wallet 202.
Providing students with ownership can teach responsibility and the secure nature of the blockchain and NFTs reduces the risk of third-parties tampering with or improperly using student data. The use of blockchain and NFT technologies accordingly allows for students to own their data in a secure and distributed manner.

Visualizing engagement insights for a student in the form of an avatar or non-fungible token (NFT) that they own promotes engagement for several reasons. First, using an avatar or NFT to represent a student's engagement data can make it more engaging and interactive. By providing a visual representation of the data, students may be more likely to pay attention to and engage with the information. Additionally, a student owning an avatar or NFT that they can customize and personalize can give the student a greater sense of ownership and control over their own learning. This can help students feel more invested in the process, which can increase their motivation and engagement. Finally, having an avatar or NFT that they own can also make the data more tangible and real for students, which can help the students see the value of the information and how it relates to their own learning.

In an embodiment, data store 108 can comprise a distributed ledger. Distributed ledger data is typically spread across multiple nodes (computational devices) on a P2P network, where each node replicates and saves an identical copy of the ledger data and updates itself independently of other nodes. Accordingly, certain inefficiencies exist for distributed ledgers, and blockchains. When a blockchain network is congested, the activity can lead to slow processing and more expensive transaction fees. Many blockchains also use a significant amount of energy.

In an example, a student user can have frequent interactions with data that can correspondingly be associated with the student's profile. In an efficiency over traditional blockchain or distributed ledger implementation, engagement module 102 can selectively instruct writes to storage. In one instance, engagement module 102 can retrieve a student's data from the distributed ledger, update the local copy of the ledger data based on engagement interactions as described herein, and selectively write to the networked data store 108. For example, a selective write can take into considerations at least one of time (e.g. after a period of activity of the user), profile (e.g. after a change to the user data is determined), or party (e.g. integrating both student data and third-party data). Accordingly, engagement module 102 can receive and integrate user data for asynchronous and selective updating to the blockchain. In this way, communications with the blockchain network are reduced, thereby lessening network congestion. Likewise, individual nodes on the network have reduced computing obligations.

With continued reference to FIG. 1, various engines of engagement module 102 can employ artificial intelligence (AI), such as an ML algorithm. Incorporation of ML algorithms to system 100 can address conventional deficiencies and technical problems associated with incorporation of AI in scaled environments, particularly considering the dynamic allocation of storage solutions as described herein.

In recent years, ML has been increasingly incorporated into applications or other computing programs to provide improved services to users. For example, ML algorithms are increasingly deployed to address challenges that are unsuitable for being, or too costly to be, addressed using traditional computer programming techniques. Increasing data volumes, widening varieties of data and more complex system requirements tend to require ML techniques. It can therefore be necessary to produce models that can analyze larger, more complex data sets and deliver faster, more accurate results and preferably without programmer intervention. In general, a ML algorithm seeks to approximate an ideal target function, f, that best maps input variables x (the domain) to output variables y (the range).

One conventional training scheme for solving ML problems includes collecting at a centralized location (e.g. a server) a plurality of training examples from a plurality of computing devices (e.g., user devices such as smartphones). A machine-learned model can then be trained at the centralized location based on the collected training examples. If a ML model is stored at a centralized location, a user computing device must send input data over the network to the server where the model is stored. The server can then apply the machine-learned model to the input data, generating inferences based on the provided information. The server can then send the resulting inference(s) to the user computing device for use.

In centralized ML scenarios, both the training examples and/or inferences must be transmitted between the user's computing device and the server via the network. This network transmission poses a risk to data security, as the transmitted data may be vulnerable to interception. Moreover, this transmission contributes to increased network traffic, potentially leading to decreased communication speeds. Furthermore, the latency associated with sending data back and forth over the network may result in delays in providing the application's services.

More recently, certain applications have included machine-learned models that are stored within the application and implemented by the application on the user device. However, this architecture is both challenging to implement and resource intensive. For example, in such a scenario, the application is required to store, manage, train, and/or implement one or more machine-learned models. Inclusion of the model and corresponding support services within the application itself can increase the data size of the application, resulting in a larger memory footprint.

These technical problems of ML are particularly felt in educational settings. Many educational institutions face the challenge of maintaining aging hardware. These devices may not have the processing power or capabilities to support sophisticated features and resource-intensive functionalities, such as ML.

In embodiments, monitoring engine 114 can apply a ML algorithm to detected behaviors associated with engagement levels. Correlations, patterns, and trends of student behaviors can be extracted manually or automatically by ML approaches such as, for example, neural networks, to produce personalized adjustments to a (e.g., each) student's personalized learning profile. Behaviors detected in particular groups of students, such as a classroom, can also be extracted to provide suggestions to improve engagement of the group. For example, if a class regularly becomes distracted at a certain time of day the ML algorithm can recognize a series of common characteristics between the students, such as the students belonging to the same class, and a common reduction in engagement, and suggest altering the class schedule to provide a break at that time.

Behaviors detected by monitoring engine 114, which can each contain a series of characteristics associated with a particular student's engagement, are stored in data store 108 for future comparisons with received data. Behavior comparisons can be accomplished by computing similarity metrics using correlation or ML regression algorithms. For example, if the similarity of received data to a previously detected behavior is above a certain threshold, (e.g., 75%, 90%, 95% or 99% similarity) the matching process can determine that the received data represents to a behavior associated with an engagement level.

In embodiments, as described above, as a student continues to interact with engagement module 102, the dataset used as a basis to inform future engagement recommendations and drive learning decisions grows. This dataset can be used for training ML models. Accordingly, as the dataset grows, the system 100 provides better and more accurate recommendations.

In embodiments, the ML algorithm can extract environmental characteristics from particular portions of received data to better compare received data to known behaviors. For example, distracted behaviors can be separated based on types of distractions (i.e., physical distractions, technological distractions, distractions caused by other students) and a type of distraction can be stored with detected behaviors. Compartmentalizing the behavior recognition analysis can improve accuracy in some circumstances by limiting the influence of outlying data (e.g., a student's prolonged lack of engagement stemming from a lack of internet access rather than continued disengagement).

ML techniques can be applied to labeled (supervised) or unlabeled (unsupervised) behavior data. Further, a classifier can receive as input parameters such as type of device (a speaker and headphones may have different parameters or sound detection capabilities for example) and type of surface the touch input is generated from. Reasons for employing such a classifier include identifying the position of a transducer relative to the housing of the user device or the number of transducers present.

In operation, the received student activity (e.g., mouse clicks, page dwell time, physically observed behavior) can be processed by the ML algorithm to benefit from contextual information for the activity. In other words, the ML algorithm can allow for processing of variable length inputs and outputs by maintaining state information over time. In one example, a user may repeatedly attempt a task if they are confused. The ML algorithm can consider the previous attempt and accordingly alter subsequent analysis of the subsequent attempts, such as reducing a threshold needed to consider later attempts as an action representing a confused behavior. In another example, if multiple activities are being received over a short window of time, trends such as the student fidgeting can be identified and more efficiently identified during a session. Thus, the context surrounding a detected behavior can contribute to personalized AI insights and allow the ML algorithm to better account for wide variations in behavior across students.

The ML algorithm can be trained to identify common student traits that correlate to behaviors and engagement levels. In one example, students belonging to a young age group, such as students aged 7 to 10 years, can be found to be more likely to exhibit behaviors related to misusing classroom resources than an older age group, such as students aged 16 to 18 years. Insights between student traits can be used to personalize which behaviors correspond to an engagement level. In cases where a student has a learning disability their behaviors can be correlated with different engagement levels than peers. Student traits can thus be compared to improve behavior detection. Additionally, student traits can be used to generate a personalized learning environment more quickly for new students based on engagement levels of similar students. As more comparisons between student traits are made, received behavior data can be more efficiently analyzed to better recognize the starting and ending points of detected behaviors.

In some embodiments, training data from self-assessments produced by assessment engine 116 can be used to evaluate the effectiveness and accuracy of the ML algorithm. Self-assessments can include one or more questions relating to detected activities to confirm whether automated evaluations are accurate. Observations from educators, guardians, and other stakeholders can also be used to evaluate ML algorithm accuracy and precision. External sources can effectively serve as calibration elements for the ML algorithm in some embodiments.

Although described with respect to behavior detection of monitoring engine 114, the ML applications and techniques described herein should be understood as applicable to other functionality of the engagement module 102 where appropriate. For example, avatar generation and refinement as achieved by visualization engine 118 can similarly benefit from identifying common student traits.

In embodiments, assessment engine 116 is configured to incorporate an ML feedback mechanism. Over time, assessments generated by assessment engine 116 can be revised to improve how data is requested from students and what data is requested, such as by updating GUI elements to not request redundant data that can be automatically parsed from previously submitted user data. The ML of assessment engine 116 can improve data aggregation and analysis, including refinement of nature language processing (NLP) applications where they are used such as for short answer responses.

In one aspect, the present disclosure provides for a device-agnostic system for generating and maintaining personalized learning profiles for students. Increasing engagement is beneficial for both business and educational outcomes. Students who feel connected to their personalized engagement profile and avatar are more likely to return to sessions and to get the most benefit from participation. Additionally, embodiments of the present disclosure improve upon conventional engagement assessment by providing scalable means of applying personalized learning profiles while allowing the students to retain ownership. The ease of incorporating personalized learning profiles can bridge the divide between students' physical and digital environments and improve data collection and progress tracking across all aspects of a student's learning path.

In embodiments visualization engine 118 includes a standard profile build and custom profile build options. The standard profile build includes preset options for avatar design and customization that can be easily implemented within educational institutions. The standard profile build can be based on standard student traits and behaviors built into engagement module 102.

Custom profile build options for educational institutions, developers, and third-party developers expand upon or replace elements of the standard profile build. Custom profile build options include the ability to upload custom traits and corresponding appearance elements (e.g., artwork) to populate based on data and automated data input for custom avatar experiences. When students choose to share their personalized learning profile and avatar with other services, elements of the standard profile build can be replaced with corresponding custom elements. For example, an avatar item corresponding to a student's interest in creativity and the arts may be represented as a paint brush in the standard profile build but automatically be converted to a wand in a fantasy-themed game environment. Association of items with engagement levels and student traits can allow flexibility and seamless transitions between applications that wish to incorporate personalized learning profiles using the dedicated API. This further allows for dynamic changes to the avatar based on learners' experiences in games and other learning environments.

A profile build toolkit can be provided for third parties to develop their own avatar experiences and behaviors according to embodiments. The profile build toolkit can include a traits library for developers to choose from or enhance when customizing the avatar experience.

One aspect of the present disclosure is a method of self-assessing and improving engagement utilizing a feedback loop of inputs used to create the avatar that can be stored on the blockchain as an NFT In embodiments, the inputs include questions or personal statements with two or more potential answers. In such embodiments, an (e.g., each) answer can be correlated to a design feature of the student's individualized avatar. In an embodiment there are 17 inputs required to fully initialize a student avatar.

Embodiments of the present disclosure provide for a learning platform configured to implement a learning profile on a blockchain. The learning profile comprises traits, an engagement profile, and an avatar associated with a student and serves to characterize the learning ability and engagement of a user. In embodiments, the learning profile can be used to connect identified behaviors of a student to an engagement framework for explaining the user's current engagement, developing a plan that will move the user to a higher level of engagement, and presenting a visual representation of user specific engagement and learning preferences.

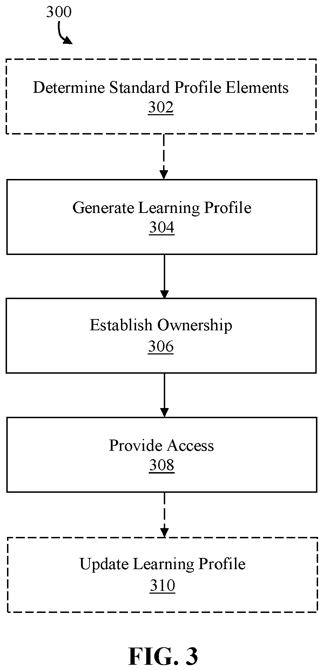

Referring to FIG. 3, a flowchart of method 300 for implementing a personalized learning profile is depicted, according to an embodiment. In an embodiment, method 300 can be implemented by system 100 as depicted in FIG. 1.

At 302, method 300 optionally comprises determining standard profile elements. Standard profile elements can include, for example, traits and engagement profiles. For example, standard profile elements can serve as baseline (e.g., preset) options as part of learning profile creation. Standard options for traits and engagement profiles to be incorporated into a personalized learning profile can provide improved data processing and reduce complexity of implementing the learning platform on participating devices. In an embodiment, student traits can include, for example, one or more of: learning style, interests, self-regulation, motivation, resilience, curiosity, social skills, cognitive abilities, and prior knowledge.

In an embodiment, standard profile elements can improve consistency and interoperability. Standardized trait and engagement profile options can ensure consistency across different learning profiles and educational institutions, facilitating interoperability and data exchange between applications and administrators. This consistency allows for seamless integration and interoperability of learning profile data across diverse educational environments.

In an embodiment, standard profile elements can improve data structuring and organization. Standardized trait options can provide a structured workflow for organizing and categorizing learning profile data, simplifying management and interpretation. By defining common traits and attributes, educational systems can efficiently store, retrieve, and process learning profile information, improving data organization and management.

In one aspect, standard profile elements improve data structuring to allow for optimization of database queries. Predefined elements facilitate the use of techniques such as query optimization, indexing, and database denormalization to improve data retrieval efficiency (e.g., minimizing database access times and improving query performance). This streamlined access is particularly useful in streamlining access to learning profile data stored via a blockchain.

In an embodiment, standard profile elements can improve integration with analytical tools. In one aspect, standardized trait options allow for the integration of learning profile data with analytical tools, algorithms, and data processing pipelines for insights generation, predictive modeling, and decision-making. By aligning trait definitions with analytical requirements, educational systems can leverage advanced analytics to extract actionable insights from learning profile data.

In an embodiment, standard profile elements can improve automation and scalability. Standardized trait and engagement profile options support automation and scalability of learning profile management processes, such as ML applications. Automated processes can streamline administrative tasks, reduce manual effort, and ensure data consistency and accuracy across large-scale educational deployments.

In one aspect, standard profile elements provide a baseline for user-based collaborative filtering. User-based collaborative filtering is an approach that identifies users who have similar preferences or behavior patterns and recommends items liked or interacted with by those similar users to the target user. By starting learning profiles from common data points, finding users with similar traits, preferences, and/or behaviors, can be made more efficient, particularly for new users of educational institutions with limited historical data.

In a second aspect, standard profile elements facilitate refinement of item-based collaborative filtering. For example, item-based collaborative filtering is an approach that identifies items that are similar to those previously liked or interacted with by the target user and recommends them based on their similarity. This approach is particularly effective for recommending niche or less popular items, as it leverages the collective preferences of users to identify relevant items (e.g., mitigating bias).

Further at 302, a standard profile build toolkit can be provided. The profile build toolkit can include a traits library for educational institutions to choose from when customizing the learning profile and avatar experience. The traits library can facilitate quality assurance and validation of learning profile data by defining clear criteria and guidelines for trait selection, description, and validation. The traits library can ensure that learning profile data meets predefined quality standards and is reliable, accurate, and meaningful for educational stakeholders. For example, use of the standard profile build toolkit can improve interpretability and transparency of learning profile data by providing clear definitions and meanings for each trait. This promotes understanding and trust among educational stakeholders, enabling informed decision-making and accountability in educational processes.

The standard profile build toolkit can include preset options for avatar design and customization that can be easily implemented within educational institutions. The standard profile build toolkit can include an appearance element associated with each respective standard student trait and/or engagement profile.

In an embodiment, the standard profile elements can be refined over time using ML algorithms (e.g., as described herein).

At 304, method 300 comprises generating a learning profile. When a student begins using the learning platform, a learning profile is created to record their educational activities, achievements, preferences, and progress. The learning profile includes trait(s), an engagement profile, and an avatar.

In an embodiment, learning profile creation can be self-specified. Administrators (e.g., teachers) and/or students can directly provide input about their preferences by self-specifying traits through surveys, questionnaires, or profile settings within the learning platform. In examples, learning profile creation can be abstracted based on a questionnaire of preferences.

In an embodiment, learning profile creation can be based on a predefined standard profile. In examples, educational institutions can opt to identify students by class or age group. For example, students belonging to a young age group, such as students aged 7 to 10 years, can be provided a set standard profile that then develops based on activity data associated with interactions and behaviors within the learning platform. In some implementations, activity data can include login frequency, time spent on different activities, completion rates for assignments or assessments, participation in discussions or forums, and other engagement metrics.

In an embodiment, the avatar associated with a learning profile serves as a visual representation of their learning profile within the educational environment. The avatar may evolve and change based on the student's educational journey, reflecting their progress, accomplishments, and personalization preferences.

At 306, method 300 comprises establishing ownership of the generated learning profile. Data from learning profiles, including traits and engagement profiles, can be collected and stored in a centralized database. This data may be anonymized and aggregated to protect learner privacy while still providing valuable insights for profile generation. For example, learning profile can be assigned a unique ID (e.g., a number) that can be used to identify the learning profile without student identifying information.

In an embodiment, measures are implemented to protect student privacy and ensure data security throughout the profile generation process. This includes obtaining consent for data collection, anonymizing sensitive information, implementing data encryption and access controls, and complying with relevant data protection regulations.

In an embodiment, the learning profile data, including updates and changes to the avatar, is stored on a blockchain network. Updates to the learning profile can be recorded as a transaction on the blockchain. The blockchain provides an immutable record of the student's educational history, ensuring that all developments to the learning profile are securely recorded and cannot be altered or tampered with retroactively. This transparency and trustworthiness enhance the credibility and reliability of the student's achievements.

In an embodiment, blockchain transactions reflect all educational progress, such as a student completing a course, earning a badge, or achieving a milestone. Utilizing blockchain technology to secure and streamline processes related to credentialing, certification, and academic record-keeping can provide tamper-proof records.

In an embodiment, blockchain transactions are limited to learning profile adjustments, allowing for a maintained record of the student's learning ability while reducing communication overhead and PoW costs.

In an embodiment, privacy-preserving model training can be provided by the learning platform. Model training can be conducted based on learning profile development local to participating devices (e.g., using local data), without the need to share raw data with the learning platform (e.g., a central server). Instead of sending raw data to a central server for training, edge devices send learning profile updates (e.g., abstracted data) to the learning platform, which can be used to refine standard profiles and/or engagement frameworks, and to determine similarities between learning profiles. Local updates to learning profiles preserves data privacy since raw data never leaves the device.

At 308, method 300 comprises provisioning access to the generated learning profile to appropriate parties.

Storage of the learning profile in a blockchain allows for decentralized access by educational stakeholders, including students, teachers, parents, and administrators. These stakeholders can access the student's learning profile and avatar updates through decentralized applications or interfaces that interact with the blockchain network. This decentralized access ensures data transparency and ownership by the student.

In an embodiment, a dedicated API implementation can cater to the unique needs, preferences, and characteristics of individual users. For example, dedicated APIs can allow educators to specify parameters or preferences based on their individual requirements for teaching. These parameters may include content preferences, customization options, filtering criteria, and other settings that tailor the API responses to match the user's needs and preferences. In such an example, a distinct dedicated API can be provided to parents that allows for access to content and customization options that are discrete from the access provided to educators.

In an embodiment, dedicated APIs are scalable and performant to accommodate varying levels of user demand and traffic. Use caching mechanisms, load balancing, and other optimization techniques, API performance and responsiveness for users accessing the system concurrently can be enhanced. For example, during periods of high traffic to the blockchain, data associated with a recent state of a learning profile can be cached at the learning platform and provided to a user (e.g., rather than providing access to historical data stored via the blockchain).

In an embodiment, learning profiles (e.g., traits, engagement profiles, and/or avatars) can be minted as NFTs. In such an embodiment, ownership of the learning profile can be divided into fractional shares, allowing multiple owners to collectively own the learning profile. Fractional ownership opens new opportunities for asset accessibility and investment diversification, between the student and a parent or guardian, enabling broader participation in asset ownership. For example, a young student can be granted partial ownership of the learning profile or associated data and as the student develops an appreciation of data and security the portion of ownership allocated to the student can increase. Certain functionality of NFT ownership, such as transferability and smart contracts can be gated based on ownership, for example, to prevent inadvertent exposure to security threats.

In an embodiment, the learning platform centralizes authentication and access control mechanisms, allowing educational institutions to enforce consistent security policies and access controls across multiple systems and applications. The learning platform can then mediate access to the blockchain and learning profiles to third party developers. This reduces the risk of inconsistent security configurations, unauthorized access, and security gaps that may occur when managing authentication separately for each system.

At 310, method 300 optionally comprises updating the learning profile, for example, based on ongoing student interactions and feedback. As students engage with the learning platform, their engagement behaviors are monitored, and their profiles can be adjusted accordingly to ensure relevance and accuracy over time.

In an embodiment, avatar customization is provided by the learning platform. Based on the student's learning profile and achievements, the avatar can be customized and updated to reflect their progress and accomplishments. For example, earning a certificate or mastering a skill may unlock new avatar features, accessories, or visual enhancements.
In an embodiment, a blockchain can facilitate updating an avatar based on historical developments of a learning profile of a student through a decentralized and immutable ledger that securely stores and tracks the student's educational progress. A dynamically updating avatar that reflects the student's learning journey enhances personalization and engagement within the educational environment. Students feel a sense of ownership and pride in ownership of their avatar's representation, fostering a positive learning experience and encouraging continued participation. By leveraging blockchain technology to securely store and track a student's educational progress via a learning profile, the learning platform can update the avatar based on historical updates to the learning profile and provide decentralized access to this information, enhancing transparency, credibility, and engagement while empowering students to take ownership of their learning journey.

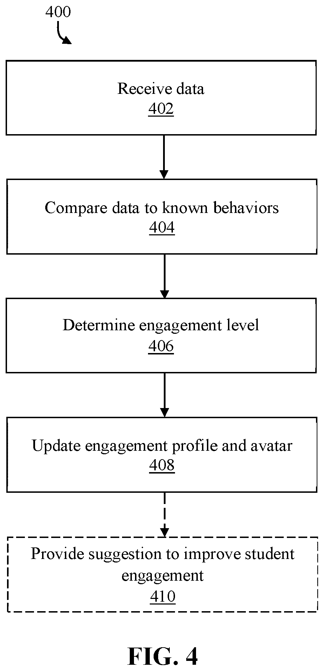

Referring to FIG. 4, a flowchart of feedback loop 400 for determining a student's engagement level is depicted, according to an embodiment.

At block 402, data is received, such as by monitoring engine 114 that describes the student's activity or by assessment module 116 that requests self-reflection on performance. This data can be manually submitted from an educator or guardian and/or automatically tracked by at least one of monitoring engine 114 or assessment module 116.

In embodiments, a GUI can prompt users to provide a set of input data. Requested input data can depend on student traits and the application of the personalized learning profile. For example, input data can vary depending on if the student is in a classroom or is interacting with a digital game incorporating the student's avatar. Requested input data can be categorized based on one or more levels of engagement (e.g., distracted, following instructions), source, and associated student traits.

At block 404, received data for the student is evaluated in relation to known behaviors. In embodiments, AI, such as an ML algorithm, can be used to automate this process for student activity data automatically collected by monitoring engine 114.

At block 406, an appropriate engagement level is determined for the student based on the identified behavior. The appropriate engagement level is determined in accordance with the student's personalized learning profile. For example, a response shared with a class by a student that traditionally refrains from active participation can be correlated to a higher engagement level than the shared response from a student who frequently shares.

Engagement levels are used to group behaviors per level, at each level there are a series of behaviors that are measured and assessed based on the level. The inputs of the profile are used as a way for a (e.g., each) student to self-assess what works for them in an educational environment.

At block 408, the student's engagement profile and avatar are updated based on the determined engagement level. In embodiments, the visual appearance of the avatar is only updated upon confirmation by the student.

At block 410, a report of behavior history can optionally be provided to the student, a guardian, or an educator. The report can include a suggestion to improve the student's engagement where applicable. In embodiments, improvement suggestions can be based on environmental or material adjustments that have a history of being successful for students with similar traits and/or students who exhibit similar behavior.

Embodiments of the present disclosure incorporate engagement as a metric for success in both digital and physical learning environments. The use of engagement levels is significant as it assists student development and a love for learning without the anxiety that is often introduced at an early age. It also allows for a more holistic approach to education that focuses on skills beyond just traditional academic subjects.

Teaching a student about themselves or helping the student develop their meta-cognitive skills, can have a positive impact on the student's engagement in learning. Meta-cognition refers to an individual's ability to think about their own thinking processes, and includes skills such as planning, monitoring, and evaluating their own learning. By developing meta-cognitive skills, a student can become more aware of their own strengths and weaknesses, as well as the strategies that are most effective for the student. This can help the student take a more active and self-directed approach to learning, which can increase their motivation and engagement. Additionally, teaching students about meta-cognition can help students develop a growth mindset, which is the belief that their abilities can be developed through effort and practice. A growth mindset has been shown to be associated with higher levels of engagement and achievement in learning. Engagement benefits are compounded when a student has ownership over the data related to their learning. This is because having ownership to one's data can give students a greater sense of agency and control over their own learning. This can further help students develop a growth mindset.

A single source for assessing and understanding engagement in both digital and physical learning environments provides a holistic view of a student's engagement. In today's world, many students engage with learning in digital and physical environments and it is important to be able to track and measure student engagement across environments. By having one source to collect and analyze engagement data from both types of environments, educators and other stakeholders can get a more complete picture of how a learner is engaging with learning and can use this information to tailor their approach to better support the learner. Additionally, having one place to assess engagement can help facilitate communication and collaboration between educators, guardians, students, and other stakeholders and can provide a common language and framework for understanding and addressing engagement issues.

In embodiments, a learning platform of the present disclosure is configured to support multiple engagement profiles for a single user. Use of multiple engagement profiles can allow for context-aware compartmentalization, improved interaction quality, and efficient data organization. For example, the learning platform can generate multiple engagement profiles for a user for different contexts, such as for in-classroom engagement or out-of-school engagement.

In an embodiment, personalized recommendations generated by the learning platform can be more accurate when they are based on the interests and behavior patterns associated with a particular context. Moreover, compartmentalization of engagement data for each engagement profile can allow the learning platform to more easily make storage allocation determinations. In some implementations, the learning platform can separately store activities and engagement data to improve privacy and security. For example, a user might want to keep their extracurricular activities separate from in-classroom activities. Moreover, by limiting, and in some cases centrally storing, the amount of personal information associated with engagement profiles, the learning platform can reduce the risk of data exposure if one engagement profile is corrupted or compromised.

In an embodiment, multiple engagement profiles can facilitate context aware behavior monitoring and communication. For instance, an engagement profile associated with in-school behavior may prioritize formal behavior (e.g., active listening and organized communication), while an engagement profile associated with social, extracurricular activities, such as third-party games, can be more casual. In such an example, the engagement profile associated with social activities can promote and/or reward behaviors that are reflective of engagement but are not appropriate for a formal education environment. Accordingly, multiple profiles can lead to increased engagement as users interact in more varied ways, depending on the context. The learning profile can capture a richer data set by observing how users interact across different profiles.

In an embodiment, a learning platform of the present disclosure is configured to dynamically switch between engagement profiles associated with a user. Determination of an active engagement profile by the learning platform can be based on, for example, user devices, user roles, locations, applications, and the like. For example, users who have multiple roles (e.g., a student and a tutor) can manage each role's needs separately, ensuring that they have the right tools and engagement information available for each.

In an embodiment, an engagement profile can be automatically activated/implemented when a user is determined to be in a location, for example, based on geographic data associated with a user device. For example, if a user is determined to be at a school a first engagement profile can be selected, otherwise a second engagement profile can be selected (e.g., if the user is at home).

In an embodiment, ML algorithms can be applied (e.g., as described herein) to refine activation logic for engagement profiles. By supporting multiple engagement profiles for a single user (e.g., learning profile), the learning platform can offer a more personalized, secure, and efficient user experience, ultimately leading to higher user satisfaction.

In embodiments, a learning platform of the present disclosure is configured to strategically allocate learning profile data between a blockchain and traditional database, for example to improve efficiency. A hybrid combination of cloud storage with decentralized storage can allow for balancing of efficiency, cost, and security.

In an embodiment, the learning platform can determine whether received data elements should be stored as on-chain data (e.g., stored in the blockchain) or off-chain data (e.g., not stored in the blockchain). On-chain data can be critical, high-integrity data that benefits from immutability, transparency, and decentralization on the blockchain. This includes transaction records (e.g., historical developments of learning profiles, including avatars), smart contract logic, and proof of ownership or provenance. Off-chain data can be large, non-critical, or frequently changing data off the blockchain in external databases. Off-chain data can include monitored data associated with student engagement/behavior, student files (e.g., individual assessments) that can lead to learning profile modifications, or sensitive information.

In an embodiment, the learning platform can implement a sidechain for administrator access. The sidechain can be used for specific modification requests and functionalities that require higher scalability or different consensus mechanisms, such as for integration of learning profiles to third-party applications. In such an embodiment, the main blockchain handles security and settlement, while the sidechain can be used to manage transactions.

In an embodiment, the learning platform can incorporate oracle services to connect on-chain and off-chain data. Oracles can fetch data from an external database associated with the learning platform and selectively provision the data to the blockchain based on allocation determinations by the learning platform. Oracles can allow for smart contracts to have access to non-sensitive portions of student data that can otherwise be associated with (e.g., stored based on) sensitive student data in the external database.

In an embodiment, the learning platform can be configured to perform data partitioning, for example, via sharding and/or data segmentation. Sharding can include dividing the blockchain into smaller, more manageable pieces referred to as shards. A (e.g., each) shard can contain a subset of the blockchain's data. This can enhance scalability and reduce the load on individual nodes.

In some implementations, the learning platform can determine to implement sharding for standard profiles. This determination can be made based on usage patterns of the standard profiles. For example, the standard profiles can be more frequently requested/accessed such that portioning the standard profiles to a separate blockchain node can be advantageous. Further, because standard profiles may be used primarily for learning profile generation (e.g., rather than ongoing updates) inefficiencies introduced from blockchain silos, such as communication or transfer of assets between the blockchain nodes, can be minimal.

In an embodiment, the learning platform can implement privacy-preserving techniques such as zero-knowledge proofs or confidential transactions. A zero-knowledge proof can allow for data verification without revealing the actual data, keeping sensitive information off-chain while still leveraging blockchain's verification capabilities. Use of confidential transaction protocols can enhance privacy for on-chain data, minimizing the amount of sensitive information stored directly on the blockchain.

The learning platform is configured to perform allocation determinations between hybrid storage solutions (e.g., user devices, cloud storage, databases, blockchains), according to embodiments. These allocation determinations can include determinations to implement one or more of the blockchain feature(s) provided herein. In an embodiment, an allocation decision is based on the nature of the data and/or the specific requirements of the application. The nature of the data can include, for example, one or more of sensitivity, size, frequency of access, frequency of updates, and compliance. For example, data subject to privacy laws that require the ability to delete or modify information can be stored off-chain to comply with regulatory requirements.

In an embodiment, data considered sensitive by the learning platform can include personal information, financial records, and proprietary information. If the learning platform determines data (e.g., user input) is sensitive, the learning platform can implement stringent access controls and privacy measures, making it more suitable for off-chain storage. Otherwise, if the learning platform determines data is public, meaning the data is intended to be transparent and publicly verifiable, such as an avatar reflective of a learning profile, the learning platform can store the public data stored on-chain.

In an embodiment, the learning platform can analyze interaction metrics (e.g., get requests, etc.) to identify high frequency and/or dynamic data. For example, the learning platform can consider (e.g., label) session information and real-time analytics as high-frequency data best suited for off-chain storage (e.g., to avoid performance bottlenecks). The learning platform can (e.g., otherwise) determine low frequency and/or static data, such as historical records and audit trails associated with learning profiles, is to be stored on-chain.

In an embodiment, the learning platform can determine whether data should be immutable. In some implementations, the learning platform can be configured with predetermined rules and/or mappings to identify data requiring a high-level of integrity. For example, the learning platform can store data that must be tamper-proof, such as audit logs, transaction records, and ownership proofs, on-chain. For example, the learning platform can store data that needs to be updated or deleted, such as user settings, operational data, and temporary records, off-chain.

Embodiments of the present disclosure accordingly provide for a learning platform configured to leverage dynamic storage allocation to significantly improve efficiency, scalability, and usability.

In an embodiment, a system for dynamic evaluation and visualization of user engagement comprises an engagement module including computing hardware of at least one processor and memory operably coupled to the at least one processor. The system can include instructions that, when executed on the engagement module, cause the engagement module to implement a monitoring engine, an assessment engine, and a visualization engine. The monitoring engine is configured to receive behavior data associated with a period of activity of a user. The assessment engine is configured to generate an assessment based on the period of activity and determine an engagement level of the user based on the behavior data and the assessment. The visualization engine is configured to generate an avatar based on the engagement level and generate a transaction on a blockchain. The transaction can indicate the user owns the avatar.

In one aspect, a system further includes a reporting engine configured to implement a dedicated API for a networked user, wherein the networked user modifies the blockchain with a new transaction, the new transaction modifying the avatar.

In one aspect, the transaction on the blockchain includes an engagement profile characterizing the learning ability of the user.

In one aspect, the assessment engine is further configured to determine the engagement level of the user based on a comparison of the behavior data to known behavior data. In examples, the known behavior data includes historical data of the user. The historical data of the user can include a dataset that grows over time as the user interacts with the engagement module.

In an embodiment, the monitoring engine, assessment engine, and/or visualization engine can be configured to implement machine learning model(s). For example, the monitoring engine can be configured to implement a machine learning model to extract the behavior data using a neural network. For example, the assessment engine can be configured to implement a machine learning model with a feedback component to generate revised behavior data.

In one aspect, a system further includes a digital wallet, wherein the avatar is minted as a non-fungible token in the digital wallet.

In an embodiment, a method for dynamic evaluation and visualization of user engagement using a blockchain comprises receiving behavior data associated with a period of activity of a user, generating an assessment based on the period of activity, determining an engagement level of the user based on the behavior data and the assessment, generating an avatar based on the engagement level, and generating a transaction on the blockchain, the transaction indicating the user owns the avatar.

In an embodiment, a data structure stored on a blockchain includes a learning engagement profile personalized to a user, the learning engagement profile characterizing a learning ability of a user, an avatar including a plurality of design features based on the learning engagement profile, wherein the learning engagement profile and the avatar are updated on the blockchain based on a user interacting with a digital environment and a physical environment.

The follow is a non-exhaustive list of numbered examples which may or may not be claimed:
Example 1.1. A system for dynamic evaluation and visualization of user engagement, the system comprising: a learning platform including a memory and at least one processor configured to: generate a learning profile associated with a user, the learning profile including at an engagement profile and an avatar, wherein the engagement profile defines at least two levels of engagement and the avatar is a visual representation of the engagement profile; create a transaction on a blockchain, the transaction including a first copy of the learning profile and an indication that the user owns the learning profile; store a second copy of the learning profile in a database; receive a modification request associated with the learning profile; and select at least one of the first copy of the learning profile or the second copy of the learning profile to modify based on the modification request and a determination of whether the modification request is associated with the user.
Example 1.2. The system of Example 1.1, wherein, based on a determination that the modification request is associated with the user, the first copy of the learning profile is selected, and wherein the modification comprises creating a second transaction on the blockchain, the second transaction including the first copy of the learning profile and an indication of a modification made to the first copy of the learning profile.
Example 1.3. The system of Example 1.1, wherein, based on a determination that the modification request is not associated with the user, the second copy of the learning profile is selected, and wherein the modification comprises updating the second copy of the learning profile in the database.
Example 1.4. The system of Example 1.3, wherein the learning platform is further configured to: receive a confirmation of the modification request by the user; and create a second transaction on the blockchain based on the confirmation, the second transaction including the first copy of the learning profile and an indication of a modification made to the first copy of the learning profile.
Example 1.5. The system of Example 1.1, wherein based on the determination that the modification request is associated with the user the first copy of the learning profile and the second copy of the learning profile are selected, wherein the modification to the first copy of the learning profile comprises creating a second transaction on the blockchain, the second transaction including the first copy of the learning profile and an indication of a modification made to the first copy of the learning profile, and wherein the modification to the second copy of the learning profile comprises updating the second copy of the learning profile in the database.
Example 1.6. The system of any one of Examples 1.1-1.5, wherein the learning platform is further configured to implement a dedicated API for the user, wherein the dedicated API for the user is configured to provide access to the first copy of the learning profile and to modify the blockchain.
Example 1.7. The system of any one of Examples 1.1-1.6, wherein the learning platform is further configured to implement a public API, wherein the public API is configured to provide access to the second copy of the learning profile.
Example 1.8. The system of any one of Examples 1.1-1.7, wherein the learning platform is further configured to: create a digital wallet associated with the user and the blockchain; and mint a non-fungible token (NFT) of the avatar in the digital wallet, wherein the digital wallet is configured to provide the user with access to the NFT.
Example 1.9. The system of any one of Examples 1.1-1.8, wherein the learning profile further includes an identifier associated with the user, wherein the learning profile consists of non-identifying information of the user and the identifier.
Example 1.10. The system of any one of Examples 1.1-1.9, wherein the learning platform is further configured to: provide an assessment; and receive, based on the assessment, user preference data indicative of at least one user preference, wherein the learning profile is generated based on the user preference data.
Example 1.11. The system of any one of Examples 1.1-1.10, wherein the learning profile further includes a trait of the user, wherein the avatar is further based on the trait.
Example 2.1. A method for dynamic evaluation and visualization of user engagement, the method comprising: receiving user preference data associated with a user; generating, based on the user preference data, a learning profile associated with the user, the learning profile including at an engagement profile and an avatar, wherein the engagement profile defines at least two levels of engagement; creating a transaction on a blockchain, the transaction including a first copy of the learning profile and an indication that the user owns the learning profile; storing a second copy of the learning profile in a database; providing a dedicated API for the user, wherein the dedicated API for the user is configured to provide access to the first copy of the learning profile and to modify the blockchain; providing a public API, wherein the public API is configured to provide access to the second copy of the learning profile; receiving a modification request associated with the learning profile; and selecting at least one of the first copy of the learning profile or the second copy of the learning profile to modify based on whether the modification request was received via the dedicated API or the public API.
Example 2.2. The method of Example 2.1, wherein the user preference data includes an indication of a selected trait from a plurality of predefined traits, wherein the avatar is based on the selected trait.
Example 2.3. The method of any one of Examples 2.1-2.2, further comprising: creating a digital wallet associated with the user and the blockchain; and minting a non-fungible token (NFT) of the avatar in the digital wallet, wherein the digital wallet is configured to provide the user with access to the NFT
Example 2.4 The method of any one of Examples 2.1-2.3, wherein the learning profile is generated further based on the a comparison of the user preference data with a predefined learning profile.

In some embodiments, the effects and/or advantages associated with the system of Examples 1.1-1.11 as discussed above are applicable in analogue to the methods of Examples 2.0-2.4.

All Examples (e.g., Examples 1.1 to 2.4) may advantageously enable the provisioning and execution of distributed storage workflows based on that data, for example, to improve security and efficiency. By selectively implementing storage operations between a learning platform and a blockchain, the Examples reduce overhead associated with data growth, increase interoperability (e.g., by counteracting blockchain siloing), and increase usability.

Various embodiments of systems, devices, and methods have been described herein. Embodiments are given only by way of example and are not intended to limit the scope of the claimed inventions. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the claimed inventions.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

Although a dependent claim may refer in the claims to a specific combination with one or more other claims, other embodiments can also include a combination of the dependent claim with the subject matter of each other dependent claim or a combination of one or more features with other dependent or independent claims. Such combinations are proposed herein unless it is stated that a specific combination is not intended.

Any incorporation by reference of documents above is limited such that no subject matter is incorporated that is contrary to the explicit disclosure herein. Any incorporation by reference of documents above is further limited such that no claims included in the documents are incorporated by reference herein. Any incorporation by reference of documents above is yet further limited such that any definitions provided in the documents are not incorporated by reference herein unless expressly included herein.

For purposes of interpreting the claims, it is expressly intended that the provisions of 35 U.S.C. § 112(f) are not to be invoked unless the specific terms "means for" or "step for" are recited in a claim.

Examples of the present disclosure are set out in the following numbered clauses.
1. A system for dynamic evaluation and visualization of user engagement, the system comprising:
   a learning platform including a memory and at least one processor configured to:
   generate a learning profile associated with a user, the learning profile including at an engagement profile and an avatar, wherein the engagement profile defines at least two levels of engagement and the avatar is a visual representation of the engagement profile;
   create a transaction on a blockchain, the transaction including a first copy of the learning profile and an indication that the user owns the learning profile;
   store a second copy of the learning profile in a database;
   receive a modification request associated with the learning profile; and
   select at least one of the first copy of the learning profile or the second copy of the learning profile to modify based on the modification request and a determination of whether the modification request is associated with the user.
2. The system of clause 1, wherein, based on a determination that the modification request is associated with the user, the first copy of the learning profile is selected, and wherein the modification comprises creating a second transaction on the blockchain, the second transaction including the first copy of the learning profile and an indication of a modification made to the first copy of the learning profile.
3. The system of clause 1, wherein, based on a determination that the modification request is not associated with the user, the second copy of the learning profile is selected, and wherein the modification comprises updating the second copy of the learning profile in the database.
4. The system of clause 1, wherein the learning platform is further configured to:
   provide a dedicated API for the user, wherein the dedicated API for the user is configured to provide access to the first copy of the learning profile and to modify the blockchain; and
   provide a public API configured to provide access to the second copy of the learning profile, wherein the determination of whether the modification request is associated with the user is based on whether the modification request is received via the dedicated API or the public API.
5. The system of clause 1, wherein the learning platform is further configured to:
   create a digital wallet associated with the user and the blockchain; and
   mint a non-fungible token (NFT) of the avatar in the digital wallet, wherein the digital wallet is configured to provide the user with access to the NFT.
6. The system of clause 1, wherein the learning platform is further configured to:
   obtain behavior data associated with a period of activity of a user;
   determine an engagement level of the user from one of the at least two levels of engagement based on the behavior data and the engagement profile; and
   send an indication of the engagement level.
7. The system of clause 6, wherein the engagement level is determined further based on a comparison of the behavior data to historical data of the user interacting with the learning platform.
8. The system of clause 6, wherein the behavior data is associated with a type of distraction, wherein the engagement level is further based on the type of distraction.
9. The system of clause 6, wherein the engagement profile is automatically updated based on a machine learning model with a feedback component incorporating the behavior data.
10. The system of clause 1, wherein the learning profile further includes an identifier associated with the user, wherein the learning profile is only associated with the user via the identifier.
11. The system of clause 1, wherein the learning platform is further configured to:
   provide an assessment; and
   receive, based on the assessment, user preference data indicative of at least one user preference, wherein the learning profile is generated based on the user preference data.
12. The system of clause 1, further comprising updating the avatar based on the modification request.
13. The system of clause 1, wherein the blockchain is configured to store a historical record of modifications to the learning profile.
14. The system of clause 1, wherein the learning platform is further configured to:
   generate a second learning profile associated with the user, wherein the learning profile is associated with a first learning environment and the second learning profile is associated with a second learning environment.
15. A method for dynamic evaluation and visualization of user engagement, the method comprising:
   receiving user preference data associated with a user;
   generating, based on the user preference data, a learning profile associated with the user, the learning profile including at an engagement profile and an avatar, wherein the engagement profile defines at least two levels of engagement;
   creating a transaction on a blockchain, the transaction including a first copy of the learning profile and an indication that the user owns the learning profile;
   storing a second copy of the learning profile in a database;
   providing a dedicated API for the user, wherein the dedicated API for the user is configured to provide access to the first copy of the learning profile and to modify the blockchain;
   providing a public API, wherein the public API is configured to provide access to the second copy of the learning profile;
   receiving a modification request associated with the learning profile; and
   selecting at least one of the first copy of the learning profile or the second copy of the learning profile to modify based on whether the modification request was received via the dedicated API or the public API.
16. The method of clause 15, the user preference data includes an indication of a selected trait from a plurality of predefined traits, wherein the avatar is based on the selected trait.
17. The method of clause 16, further comprising:
   creating a digital wallet associated with the user and the blockchain; and
   minting a non-fungible token (NFT) of the avatar in the digital wallet, wherein the digital wallet is configured to provide the user with access to the NFT
18. The method of clause 9, further comprising:
   obtaining behavior data associated with a period of activity of a user;
   determining an engagement level of the user from one of the at least two levels of engagement based on the behavior data and the engagement profile; and
   sending an indication of the engagement level.
19. The method of clause 18, further comprising:
   updating the engagement profile based on a machine learning model with a feedback component incorporating the behavior data.
20. A system for dynamic evaluation and visualization of user engagement, the system comprising:
   a learning platform including a memory and at least one processor configured to:
   receive user preference data associated with a user;
   generate, based on the user preference data, a learning profile associated with the user, the learning profile including at an engagement profile and an avatar, wherein the engagement profile defines at least two levels of engagement;
   create a transaction on a blockchain, the transaction including a first copy of the learning profile and an indication that the user owns the learning profile;
   store a second copy of the learning profile in a database;
   obtain behavior data associated with a period of activity of a user;
   determine an engagement level of the user from one of the at least two levels of engagement based on the behavior data and the engagement profile; and
   generate a recommendation to improve engagement based on the determined engagement level.

## Claims

1. A system for dynamic evaluation and visualization of user engagement, the system comprising:
an engagement module including computing hardware of at least one processor and memory operably coupled to the at least one processor; and
instructions that, when executed on the engagement module, cause the engagement module to implement:
a monitoring engine configured to receive behavior data associated with a period of activity of a user;
an assessment engine configured to generate an assessment based on the period of activity, and determine an engagement level of the user based on the behavior data and the assessment; and
a visualization engine configured to generate an avatar based on the engagement level, and generate a transaction on a blockchain, the transaction indicating the user owns the avatar.

2. The system of claim 1, further comprising:
a reporting engine configured to implement a dedicated API for a networked user, wherein the networked user modifies the blockchain with a new transaction, the new transaction modifying the avatar.

3. The system of claim 1, wherein the transaction on the blockchain includes an engagement profile characterizing the learning ability of the user.

4. The system of claim 1, wherein the assessment engine is further configured to determine the engagement level of the user based on a comparison of the behavior data to known behavior data, optionally wherein the known behavior data comprises historical data of the user, the known behavior data including a dataset that grows over time as the user interacts with the engagement module.

5. The system of claim 1, wherein the monitoring engine is further configured to implement a machine learning model to extract the behavior data using a neural network.

6. The system of claim 1, further comprising a digital wallet, wherein the avatar is minted as a non-fungible token in the digital wallet.

7. The system of claim 1, wherein the assessment engine is further configured to implement a machine learning model with a feedback component to generate revised behavior data.

8. A method for dynamic evaluation and visualization of user engagement using a blockchain, comprising:
receiving behavior data associated with a period of activity of a user;
generating an assessment based on the period of activity;
determining an engagement level of the user based on the behavior data and the assessment;
generating an avatar based on the engagement level; and
generating a transaction on the blockchain, the transaction indicating the user owns the avatar.

9. The method of claim 8, further comprising reporting the transaction to a networked user, wherein the networked user modifies the blockchain with a new transaction, the new transaction modifying the avatar.

10. The method of claim 8, wherein the transaction on the blockchain includes an engagement profile characterizing the learning ability of the user.

11. The method of claim 8, further comprising determining the engagement level of the user based on a comparison of the behavior data to known behavior data.

12. The method of claim 11, wherein the known behavior data comprises historical data of the user, the known behavior data including a dataset that grows over time as the user interacts over a plurality of periods of activity, the method optionally further comprising implementing a machine learning model to extract the behavior data using a neural network.

13. The method of claim 8, further comprising:
providing a digital wallet; and
minting the avatar as a non-fungible token in the digital wallet.

14. The method of claim 8, further comprising implementing a machine learning model with a feedback component to generate revised behavior data.

15. A data structure stored on a blockchain including:
a learning engagement profile personalized to a user, the learning engagement profile characterizing a learning ability of a user;
an avatar including a plurality of design features based on the learning engagement profile,
wherein the learning engagement profile and the avatar are updated on the blockchain based on a user interacting with a digital environment and a physical environment.
